# EUROPEAN PATENT APPLICATION

(11) **EP 4 488 395 A2**
(43) Date of publication of application: **08.01.2025**
(21) Application number: 24211918.8
(22) Date of filing: 05.06.2020
(51) Int. Cl.: C22C 1/05

(54) **PROXIMITY-BASED LABELING SYSTEMS AND APPLICATIONS THEREOF**

(30) Priority: 07.06.2019 US 201962858539 P; 27.02.2020 US 202062982576 P
(62) Divisional of application: 20819012.4
(71) Applicant: The Trustees of Princeton University, Princeton, NJ 08544 (US); MRL Cambridge Esc, Cambridge, MA 02141 (US)
(72) Inventor: The designation of the inventor has not yet been filed
(74) Representative: Greenwoods

(57) **Abstract**

In one aspect, compositions and methods are described herein for providing a microenvironment mapping platform operable to selectively identify various features, including protein-protein interactions on cellular membranes. In some embodiments, a composition comprises a catalyst, and a protein labeling agent, wherein the catalyst activates the protein labeling agent to a reactive intermediate. The catalyst, in some embodiments, can have electronic structure for permitting energy transfer to the protein labeling agent to form the reactive intermediate. The reactive intermediate reacts or crosslinks with a protein or other biomolecule within the diffusion radius of the reactive intermediate.

## Description

### RELATED APPLICATION DATA

The present application claims priority pursuant to Article 8 of the Patent Cooperation Treaty to United States Provisional Patent Application Serial Number 62/858,539 filed June 7, 2019 and United States Provisional Patent Application Serial Number 62/982,576 filed February 27, 2020, each of which is incorporated herein by reference in its entirety.

### FIELD

The present invention relates to proximity-based labeling techniques and, in particular, to compositions and methods permitting high resolution labeling of proteins in cellular environments.

### BACKGROUND

Protein proximity labeling has emerged as a powerful approach for profiling protein inter-action networks. The ability to label associated or bystander proteins through proximity labeling can have important implications on further understanding the cellular environment and biological role of a protein of interest. Current proximity labeling methods all involve the use of enzyme-based generation of reactive intermediates that label neighboring proteins on a few select amino acid residues through diffusion or physical contact. Despite the transformative impact of this technology, the inherent stability of these reactive intermediates such as phenoxy radicals (t_{1/2} > 100 µs) through peroxidase activation or biotin-AMP (t_{1/2} > 60 s) through biotin ligases can promote diffusion far from their point of origin. As a result, these enzyme-generated reactive intermediates pose a challenge to profiling within tight micro-environments. Furthermore, the large enzyme size, the dependency on certain amino acids for labeling, and the inability to temporally control these labeling systems present additional challenges for profiling within confined spatial regions. Given these limitations, new approaches for proximity-based labeling are needed.

### SUMMARY

In one aspect, compositions and methods are described herein for providing a microenvironment mapping platform operable to selectively identify various features, including protein-protein interactions on cellular membranes. In some embodiments, a composition comprises a catalyst, and a protein labeling agent, wherein the catalyst activates the protein labeling agent to a reactive intermediate. The catalyst, in some embodiments, can have electronic structure for permitting energy transfer to the protein labeling agent to form the reactive intermediate. The reactive intermediate reacts or crosslinks with a protein or other biomolecule within the diffusion radius of the reactive intermediate. If a protein or other biomolecule is not within the diffusion radius, the reactive intermediate is quenched by the surrounding aqueous or aqueous-based environment. As described further herein, the diffusion radius of the reactive intermediate can be tailored to specific microenvironment mapping considerations, and can be limited to the nanometer scale. In some embodiments, for example, the diffusion radius can be less than 10 nm or less than 5 nm. Moreover, in some embodiments, the reactive intermediate can have a half-life of less than 5 ns. In some embodiments, a protein labeling agent can be functionalized with a marker, such as biotin or luminescent markers for aiding in analysis. Any catalyst operable to participate in energy transfer with the protein labeling agent to provide the reactive intermediate can be employed. In some embodiments, transition metal catalyst is used. Alternatively, non-transition metal organocatalyst may be used. Energy transfer from the catalyst to the protein labeling agent can occur via a variety of mechanisms described further herein, including Dexter energy transfer.

In another aspect, conjugates for proximity-based labeling are described herein. A conjugate comprises a catalyst coupled to a biomolecular binding agent. The catalyst can have electronic structure for energy transfer to a protein labeling agent for generation of a reactive intermediate as described above. The biomolecular binding agent, in some embodiments, can be used to selectively locate or target the catalyst to a specific environment for mapping. The biomolecular binding agent, for example, locate the catalyst in the desired cellular environment for proximity labeling and associated analysis. The biomolecular binding agent can comprise a protein, polysaccharide, nucleic acid, or lipid, in some embodiments. In some instances, the biomolecular binding agent can comprise a multivalent display system comprising a protein, polysaccharide, nucleic acid, or lipid. Moreover, the biomolecular binding agent can also be a small molecule ligand with a specific binding affinity for a target protein.

In another aspect, systems for proximity-based labeling are described herein. A system, in some embodiments, comprises a conjugate including a catalyst coupled to a biomolecular binding agent, and a protein labeling agent activated by the catalyst for binding to a protein. The conjugate and protein labeling agent can have any composition and/or properties described above and in the following detailed description.

In a further aspect, methods of proximity-based labeling are described herein. A method of proximity-based labeling comprises providing a catalyst, and activating a protein labeling agent to a reactive intermediate with the catalyst. The reactive intermediate couples or bonds to a protein. In some embodiments, the catalyst is coupled to a biomolecular binding agent to selectively locate or target the catalyst to a specific environment for protein mapping in conjunction with the protein labeling agent. The catalyst, conjugate, and protein labeling agent can have composition and/or properties described above and in the following detailed description.

These and other embodiments are further described in the following detailed description.

### BRIEF DESCRIPTION OF THE DRAWINGS

FIG. 1 illustrates energy transfer between an iridium photocatalyst of Formula (I) and a diazirine protein labeling agent, according to some embodiments described herein.
FIG. 2 illustrates various diazirines operable to form reactive intermediates via energy transfer from a transition metal photocatalyst for protein or other biomolecule labeling, according to some embodiments.
FIG. 3 illustrates a reaction mechanism for flavin-based protein labeling according to some embodiments.
FIG. 4 illustrates various transition metal photocatalysts of Formula (I) having a reactive functionality for coupling a biomolecular binding agent.
FIG. 5 illustrates several transition metal photocatalysts of Formula (I) forming a conjugate with a biomolecular binding agent according to some embodiments.
FIG. 6A illustrates operation of proximity-based labeling systems and methods described herein according to some embodiments.
FIG. 6B illustrates operation of proximity-based labeling systems and methods described herein for identification and validation of membrane G protein-coupled receptors (GPCR) according to some embodiments.
FIG. 7A illustrates labeling of proteins with carbenes generated via diazirine sensitization with photocatalyst according to some embodiments.
FIG. 7B illustrates the Ir-photocatalyst employed in the reaction scheme of FIG. 7A.
FIG. 8A illustrates conjugate targeted proximity-based labeling of VEGFR2-Fc or EGFR-Fc on agrose beads showing spatially selective protein labeling of VEGFR2-Fc or EGFR-Fc.
FIG. 8B illustrates non-selectivity of primary antibody targeted labeling of agrose bead bound VEGFR2 or EGFR with HRP-secondary antibody conjugates.
FIG. 9A illustrates CD45-targeted µMapping on Jurkat cells and associated Western blot analysis.
FIG. 9B illustrates µMapping of CD45, CD29, or CD47 on Jurkat cells resulting in the enrichment of unique sets of proteins, which included both known (CD29:CD49D, CD45:CD45AP:CD2) and previously unknown interactors.
FIG. 9C illustrates results from state-of-the-art peroxidase proximity-based labeling methods where cell surface CD45 and associated proteins were not resolved from CD29 or CD47.
FIG. 10A illustrates use of conjugates and protein labeling agents described herein for µ-Mapping the PD-L1 microenvironment according to some embodiments.
FIG. 10B illustrates string analysis of convergently enriched proteins resulting from µ-Mapping the PD-L1 microenvironment, according to some embodiments.
FIG. 11A illustrates *trans* or *cis* labeling with compositions and methods described herein via intra/extrasynaptic targeting according to some embodiments.
FIG. 11B is flow cytometry analysis of antibody targeting of PD-L1 on JY-PD-L1 B cells with µMapping compositions described herein using 10-minute light irradiation relative to peroxidase-based targeted labeling according to some embodiments.
FIG. 11C is flow cytometry analysis of antibody targeting of CD45RO on Jurkat T cells with µMapping compositions described herein using 10-minute light irradiation relative to peroxidase-based targeted labeling according to some embodiments.
FIG. 11D provides two cell system confocal microscopy images illustrating selective labeling by µMapping compositions described herein relative to non-selective peroxidase-based targeted labeling according to some embodiments.
FIG. 12A illustrates bead-based protein labeling employing a secondary antibody flavin conjugate according to some embodiments.
FIG. 12B is Western blot analysis of light dependent CD45 biotinylation for the indicated time points, according to some embodiments.
FIG. 12C is a schematic of CD45RO- or CD45RA-targeted cell labeling in mixed T-cell populations according to some embodiments.
FIG. 12D is flow cytometry analysis of photolabeling time course of CD45RA+ or CD45RO+ T cell biotinylation, according to some embodiments.
FIG. 13A is a schematic depicting photoproximity labeling of CD45 on Jurkat cells with secondary antibody flavin conjugate (AFC) according to some embodiments.
FIG. 13B is Western blot analysis of CD45-targeted labeling of the Jurkat cells of FIG. 13A.
FIG. 13C is confocal imaging of cells with CD45-directed labeling indicating that biotinylation is both confined to the cell surface and light exposure time dependent.
FIG. 13D provides volcano plots of significance vs. fold-enrichment for targeted- vs isotype-targeted biotinylation of CD45 in Jurkat cells.
FIG. 14A is a schematic depicting photoproximity labeling of PDL1 on JY-PDL1 cells with secondary antibody flavin conjugate (AFC) according to some embodiments.
FIG. 14B provides volcano plots of significance vs. fold-enrichment for targeted- vs isotype-targeted biotinylation of PDL1 on Raji cells expressing PDL1.
FIG. 14C is a Venn diagram of significantly enriched proteins identified from PDL1 targeted labeling on JY and Raji cells expressing PDL1.
FIG. 14D is a list of significantly enriched proteins identified from PDL1 targeted on JY and Raji cells with known PDL1 related function.
FIG. 14E is a string protein interaction network and GO term analysis of significantly enriched proteins for PDL1-targeted experiments.
FIG. 15A is a schematic depicting a two-cell system consisting of engineered Jurkat and Raji cells according to some embodiments.
FIG. 15B is flow cytometry analysis wherein biotinylation is detected on both Raji and Jurkat cells with PDL1 targeting using the antibody flavin conjugate (AFC), but not detected using Isotype targeting or in the absence of visible light irradiation.
FIG. 15C is confocal microscopy imaging of the Raji-Jurkat two cell system with PDL1 targeting on Raji cells revealing labeling on both Raji cells and points of cellular contact on Jurkat cells using the AFC.
FIG. 15D is a schematic depicting a two-cell system consisting of engineered Jurkat and Raji cells according to some embodiments.
FIG. 15E is flow cytometry analysis wherein targeting labeling of CD45RO on Jurkat cells (known to be excluded from the synapse) resulted in low levels of Raji transcellular labeling using an AFC and nearly quantitative labeling when HRP was used.
16 illustrates the experimental set up and results, including the sulfonamide-iridium conjugate and biotin-tagged diazirine, for selective labeling of carbonic anhydrase with small-molecule based conjugates according to some embodiments.
FIG. 17 details the ability of the ligand-iridium conjugates to label their corresponding protein targets by their selectivity versus BSA according to some embodiments.
FIG. 18 illustrates identification of protein-protein interaction with proximity-based labeling compositions and methods described herein, according to some embodiments.
FIG. 19 illustrates identification of protein-protein interaction with proximity-based labeling compositions and methods described herein, according to some embodiments.
FIG. 20 illustrates identification of protein-protein interaction with proximity-based labeling compositions and methods described herein, according to some embodiments.
FIG. 21 illustrates an A₂ₐR-Ir conjugate and A₂ₐR-diazirine conjugate according to some embodiments.
FIG. 22 illustrates a photocatalytic-labeling method described herein showing a 3 log₂ fold change enrichment for ADORA2A and lack of statistical enrichment when using the stoichiometric diazirine, ADORA2A.
FIG. 23 illustrates an hGPR40-Ir conjugate and Diaz-PEG3-Bt according to some embodiments.
FIGS. 24A and 24B are Westen blot results of hGPR40 labeling by compositions and methods described herein, according to some embodiments.

### DETAILED DESCRIPTION

Embodiments described herein can be understood more readily by reference to the following detailed description and examples and their previous and following descriptions. Elements, apparatus and methods described herein, however, are not limited to the specific embodiments presented in the detailed description and examples. It should be recognized that these embodiments are merely illustrative of the principles of the present invention. Numerous modifications and adaptations will be readily apparent to those of skill in the art without departing from the spirit and scope of the invention.

### Definitions

The term "alkyl" as used herein, alone or in combination, refers to a straight or branched saturated hydrocarbon group optionally substituted with one or more substituents. For example, an alkyl can be C₁ - C₃₀ or C₁ - C₁₈ .

The term "aryl" as used herein, alone or in combination, refers to an aromatic monocyclic or multicyclic ring system optionally substituted with one or more ring substituents.

The term "heteroaryl" as used herein, alone or in combination, refers to an aromatic monocyclic or multicyclic ring system in which one or more of the ring atoms is an element other than carbon, such as nitrogen, boron, oxygen and/or sulfur.

The term "heterocycle" as used herein, alone or in combination, refers to an mono- or multicyclic ring system in which one or more atoms of the ring system is an element other than carbon, such as boron, nitrogen, oxygen, and/or sulfur or phosphorus and wherein the ring system is optionally substituted with one or more ring substituents. The heterocyclic ring system may include aromatic and/or non-aromatic rings.

The term "alkoxy" as used herein, alone or in combination, refers to the moiety RO-, where R is alkyl, alkenyl, or aryl defined above.

The term "halo" as used herein, alone or in combination, refers to elements of Group VIIA of the Periodic Table (halogens). Depending on chemical environment, halo can be in a neutral or anionic state.

Terms not specifically defined herein are given their normal meaning in the art.

### I. Proximity-Based Labeling Compositions

Compositions are described herein for providing microenvironment mapping platforms operable to selectively identify various features, including protein-protein interactions on cellular membranes. In some embodiments, a composition comprises a catalyst, and a protein labeling agent, wherein the catalyst activates the protein labeling agent to a reactive intermediate. The catalyst, in some embodiments, can have electronic structure for permitting energy transfer to the protein labeling agent to form the reactive intermediate. In some embodiments, for example, the catalyst engages in Dexter energy transfer with the protein labeling agent. The energy transfer can proceed via single electron transfer, in some embodiments.

The energy transfer to the protein labeling agent can originate from an excited state of the catalyst electronic structure, in some embodiments. The excited state of the catalyst, for example, can be a singlet excited state or triplet excited state. The excited state of the catalyst can be generated by one or more mechanisms, including energy absorption by the catalyst. In some embodiments, the catalyst is a photocatalyst, wherein the excited state is induced by absorption of one or more photons. In other embodiments, the catalyst may be placed in an excited state by interaction with one or more chemical species in the surrounding environment. Alternatively, the energy transfer to the protein labeling agent, including electron transfer, may originate from a ground state of the catalyst electronic structure.

Energy transfer, including electron transfer, to the protein labeling agent forms a reactive intermediate of the protein labeling agent. The reactive intermediate reacts or crosslinks with a protein or other biomolecule within the diffusion radius of the reactive intermediate. If a protein or other biomolecule is not within the diffusion radius, the reactive intermediate is quenched by the surrounding aqueous or aqueous-based environment. The diffusion radius of the reactive intermediate can be tailored to specific microenvironment mapping (proximity-based labeling) considerations, and can be limited to the nanometer scale. In some embodiments, for example, the diffusion radius of the reactive intermediate can be less than 10 nm, less than 5 nm, less than 4 nm, less than 3 nm, or less than 2 nm prior to quenching in an aqueous environment. Accordingly, the reactive intermediate will react or crosslink with a protein or other biomolecule within the diffusion radius or be quenched by the aqueous environment if no protein or biomolecule is present. In this way, high resolution of the local environment can be mapped via concerted effort between the catalyst and protein labeling agent. Additionally, the reactive intermediate can exhibit a t_{1/2} less than 5 ns, less than 4 ns, or less than 2 ns prior to quenching, in some embodiments. In additional embodiments, the diffusion radius can be extended to between 5-500 nm though extension of the reactive intermediate half-life.

Any catalyst-protein labeling agent combination exhibiting the foregoing electronic structure properties for energy transfer and reactive intermediate generation and associated protein or biomolecule binding can be employed for microenvironment mapping. In some embodiments, the catalyst is a transition metal complex. The transition metal complex, in some embodiments, can exhibit a long-lived triplet excited state (T₁) facilitating energy transfer to the protein labeling agent. The T₁ state can have t_{1/2} of 0.2-2 µs, for example. Transition metal complexes described herein can be photocatalytic and, in some embodiments, absorb light in the visible region of the electromagnetic spectrum. Absorption of electromagnetic radiation can excite the transition metal complex to the S₁ state followed by quantitative intersystem crossing to the T₁ state. The transition metal catalyst can subsequently undergo short-range Dexter energy transfer to a protein labeling agent, and returned to the ground state, S₀. The energy transfer to the labeling agent activates the labeling agent for reaction with a protein or other biomolecule. The T₁ state of the transition metal complex can be greater than 60 kcal/mol, in some embodiments. The metal center, for example, can be selected from transition metals of the platinum group. The metal center can be iridium, in some embodiments.

The transition metal complex can have any composition and structure consistent with the foregoing principles of excitation and energy transfer to a protein labeling agent. In some embodiments, a photocatalytic transition metal complex is hexacoordinate. Transition metal photocatalyst of proximity-based labeling compositions described herein, in some embodiments, are of Formula (I): 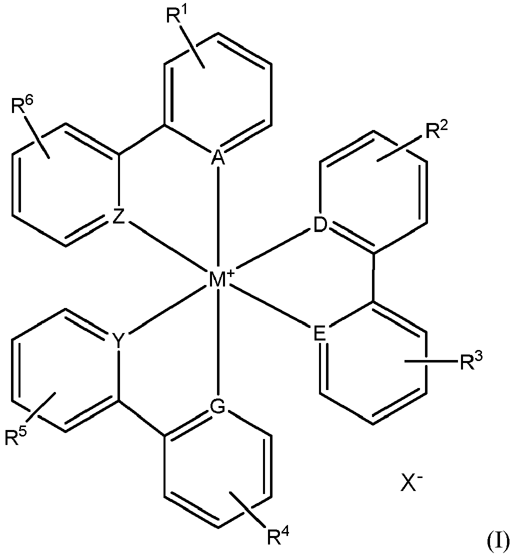
wherein M is a transition metal;
wherein A, D, E, G, Y and Z are independently selected from C and N;
wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
wherein X⁻ is a counterion. It is understood that hydrogen occupies positions on the aryl rings of Formula (I) in the absence of optional substituents R¹ - R⁶. In some embodiments, counterion (X⁻) can be selected from tetraalkylborate, tetrafluoroborate, tetraphenylborate, PF₆⁻, and chloride. In some embodiments, M is selected from transition metals of the platinum group. The metal center can be iridium.

Ligands of the transition metal complex of Formula (I) can comprise one or more pyridine moieties, in some embodiments. In some embodiments, one or more ligands comprise bipyridine or derivatives thereof. Ligands of the transition metal complex can also be selected from the species provided in Table I.

**Table I - Photocatalytic Transition Metal Complex Ligands**

| |
|---|
| dtbbpy |
| dF(CF₃)ppy |
| diPh-bpy |
| diOMe-bpy |

In some embodiments, the photocatalytic transition metal complex of Formula (I) is _{[}Ir(dF(CF₃)ppy)₂(dtbbpy)](PF₆), [Ir(dF(CF₃)ppy)₂(bpy)](PF₆), or derivatives thereof.

Additionally, ligands of photocatalytic transition metal complexes described herein, including complexes of Formula (I), can be modified with one or more polar or hydrophilic moieties for enhancing solubility of the transition metal complexes in aqueous or aqueous-based environments. Suitable moieties can include, but are not limited to, carboxyl, hydroxyl, and/or alkylene oxide moieties, such as polyethylene glycol.

As described further herein, transition metal complexes can incorporate a reactive functionality for coupling a biomolecular binding agent. In some embodiments, for example, a transition metal catalyst, including transition metal photocatalyst of Formula (I), can comprise one or more click chemistry moieties including, but not limited to, BCN (bicyclononyne, bicyclo[6.1.0]nonyne), DBCO (dibenzo-bicyclo-octyne), TCO (trans-cyclooctene), tetrazine, alkyne, and azide.

Catalyst operable for energy transfer to a protein labeling agent for producing a reactive intermediate can comprise non-transition metal catalyst, in some embodiments. Various organocatalyst for use in proximity-based labeling compounds and methods described herein can include a thioxanthone, phenothiazine, flavin, phenoxazine, benzophenothiazine, coumarin, acetophenone, or a benzophenone group, in some embodiments. In some cases, an organocatalyst comprises a triarylmethane group, rose Bengal, porphyrin, chlorin, bacteriochlorin, methylene blue, an acridine dye, a xanthene dye, or an arylmethane dye. The non-transition metal organocatalyst can have any electronic structure and/or other properties of transition metal complexes described herein, including singlet and/or triplet excited states for energy transfer to the protein labeling agent. Energy transfer from the organocatalyst to the protein labeling agent may occur via any mechanism described herein, such as Dexter energy transfer or single electron transfer. In some embodiments, an organocatalyst is a photocatalyst, wherein the excited state for energy transfer to the protein labeling agent is generated by absorption of one or more photons by the organocatalyst. Organocatalyst, for example, may absorb light in the visible region of the electromagnetic spectrum.

Protein labeling agents receive energy transfer from the catalyst to form a reactive intermediate. The reactive intermediate reacts or crosslinks with a protein or other biomolecule within the diffusion radius of the reactive intermediate. Diffusion radii of reactive intermediates are described above. Specific identity of a protein labeling agent can be selected according to several considerations, including identity of the catalyst, the nature of the reactive intermediate formed, lifetime and diffusion radius of the reactive intermediate.

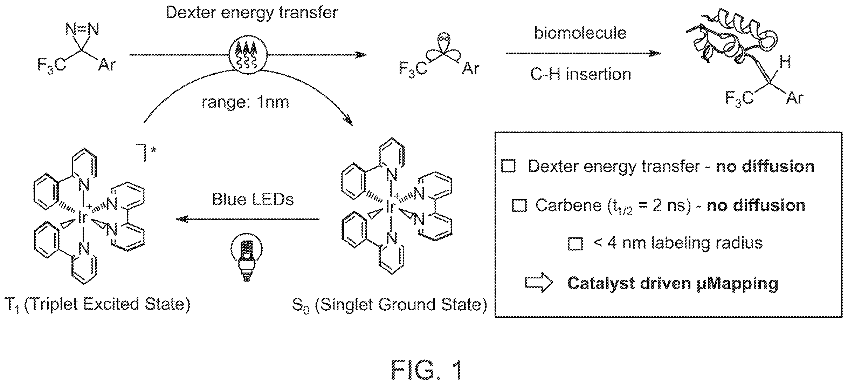

For example, in embodiments wherein the catalyst is a transition metal photocatalyst, the protein labeling agent can be a diazirine. Triplet energy transfer from the excited state photocatalyst can promote the diazirine to its triplet (T₁) state. The diazirine triplet under-goes elimination of N₂ to release a free triplet carbene, which undergoes picosecond-timescale spin equilibration to its reactive singlet state (t_{1/2} < 1 ns) which either crosslinks with a nearby protein or is quenched in the aqueous environment. FIG. 1 illustrates energy transfer between an iridium photocatalyst of Formula (I) and a diazirine protein labeling agent, according to some embodiments described herein. Notably, the electromagnetic radiation employed to generate the excited state of the transition metal catalyst does not activate the diazirine to a reactive carbene. In some embodiments, the extinction coefficient of the transition metal complex is 3 to 5 orders of magnitude greater than that of the diazirine.

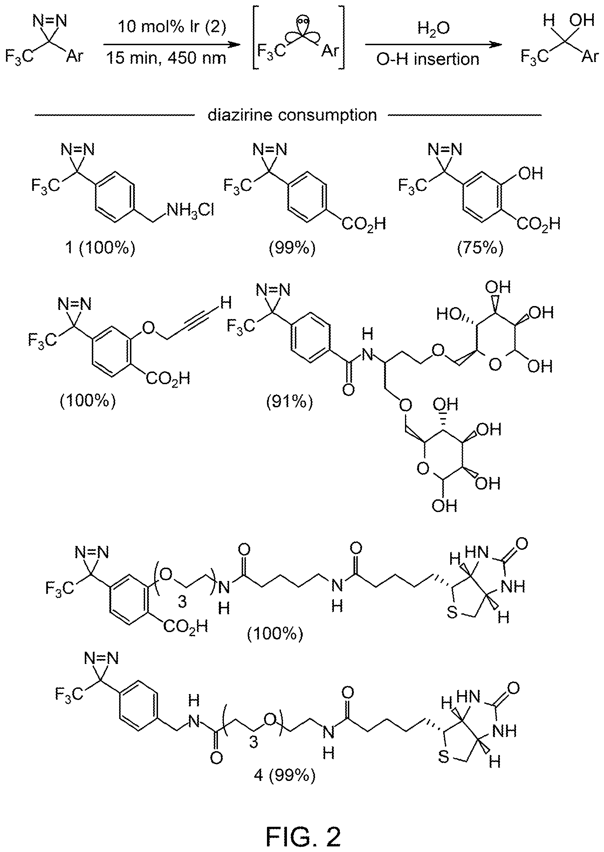

Any diazirine consistent with the technical principles discussed herein, including the reaction mechanism of FIG. 1, can be used. Diazirine sensitization, for example, can be extended to a variety of *p-* and *m*-substituted aryltrifluoromethyl diazirines bearing valuable payloads for microscopy and proteomics applications, including free carboxylic acid, phenol, amine, alkyne, carbohydrate, and biotin groups. FIG. 2 illustrates various diazirines operable to form reactive intermediates via energy transfer from a transition metal photocatalyst for protein or other biomolecule labeling, according to some embodiments. As illustrated in FIG. 2, the diazirine can be functionalized with a marker, such as biotin. In some embodiments, the marker is desthiobiotin. The marker can assist in identification of proteins labeled by the protein labeling agent. The marker, for example, can be useful in assay results via western blot and/or other analytical techniques. Markers can include alkyne, azide, FLAG tag, fluorophore, and chloroalkane functionalities, in addition to biotin and desthiobiotin.

In some embodiments wherein the catalyst is a transition metal photocatalyst, the protein labeling agent can be an azide. Triplet energy transfer from the excited state photocatalyst can promote nitrene formation from the azide. The reactive nitrene either crosslinks with a nearby protein or is quenched in the aqueous environment. Any azide operable to undergo energy transfer with eth transition metal photocatalyst for nitrene formation can be employed. In some embodiments, an azide is an aryl azide.

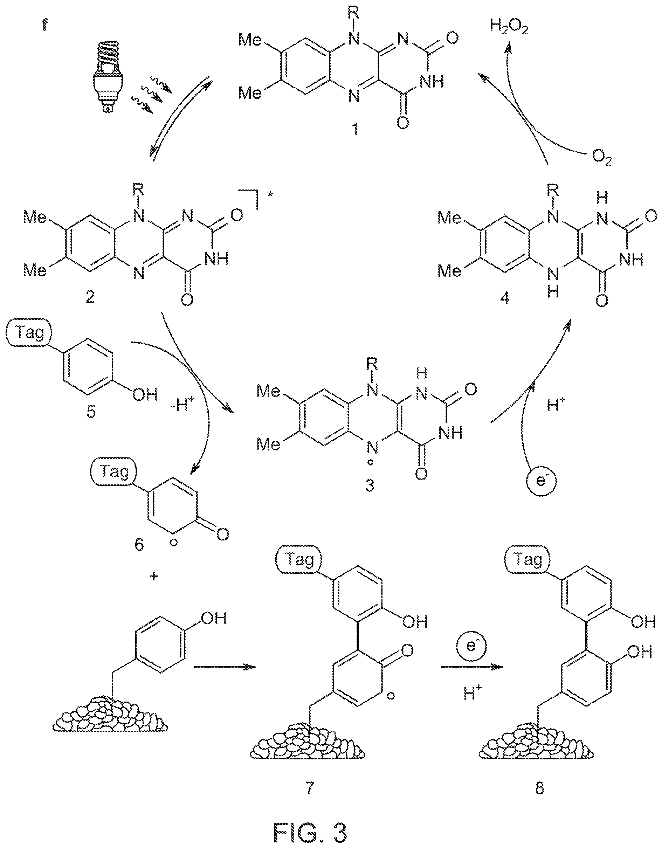

In some embodiments wherein the catalyst is an organocatalyst, the protein labeling agent can comprise one or more moieties for receiving single electron transfer from the organocatalyst. Single electron transfer to the protein labeling agent can generate a reactive radical having diffusion radius and/or lifetime described herein for reacting with a protein in the local environment of the organocatalyst. For example, an organocatalyst can be a flavin photocatalyst. The excited flavin photocatalyst can undergo single electron transfer with phenol moieties to generate a reactive phenoxy radical. FIG. 3 illustrates a reaction mechanism for flavin-based protein labeling according to some embodiments. As illustrated in FIG. 3, the phenol protein labeling agent is functionalized with a tag or marker for assisting in identification of proteins labeled by the protein labeling agent.

### II. Conjugates

In another aspect, conjugates for proximity-based labeling are described herein. A conjugate comprises a catalyst coupled to a biomolecular binding agent. The catalyst coupled to the biomolecular binding agent can comprise any catalyst described herein, including the transition metal catalysts and organocatalysts detailed in Section I above. Moreover, the biomolecular binding agent can comprise a protein, polysaccharide, nucleic acid, or lipid, in some embodiments. In some instances, the biomolecular binding agent can comprise a multivalent display system comprising a protein, polysaccharide, nucleic acid, or lipid. In some embodiments, the biomolecular binding agent can be a small molecule ligand with a specific binding affinity for a target protein. The biomolecular binding agent can be employed to locate the catalyst in the desired extracellular environment for proximity labeling and associated analysis. Accordingly, specific identity of the biomolecular binding agent can be selected according to the chemical and/or steric requirements of the desired target site for placement of the catalyst in the proximity based labeling process. Any biomolecular target site can be chosen, and target sites are not limited in the present disclosure. In some embodiments, target sites can be proteins for studying protein-protein interactions, including interaction with cellular membrane receptors. In some embodiments, for example, the biomolecular binding agent is an antibody, such as a secondary antibody for interacting with a primary antibody bound to the desired antigen. In other embodiments, the biomolecular binding agent is a ligand with specificity for a protein receptor of the cellular membrane, such as a G protein-coupled receptor.

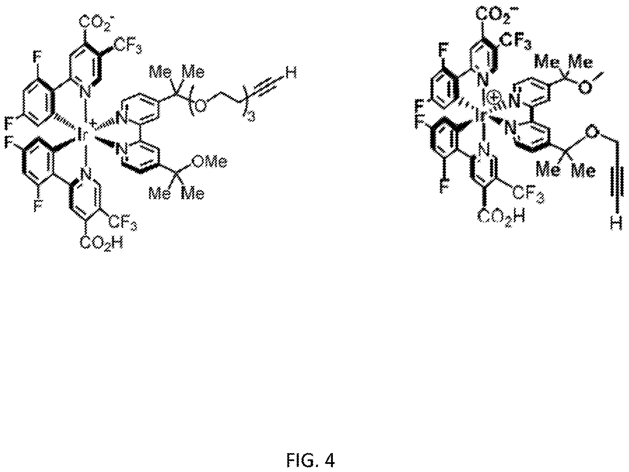

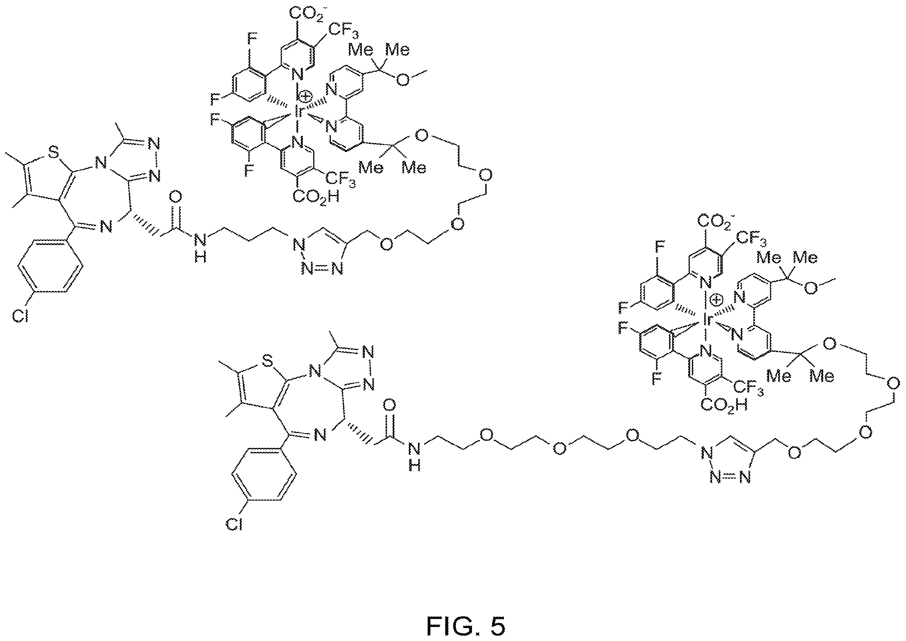

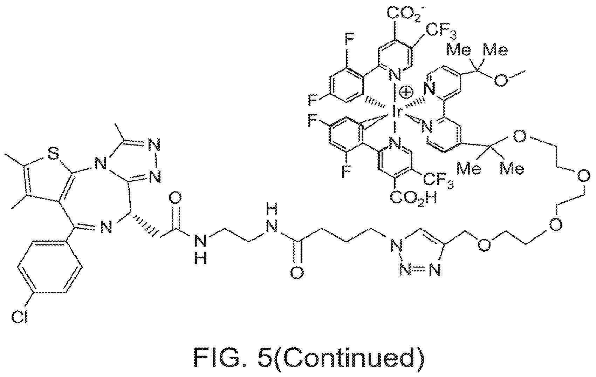

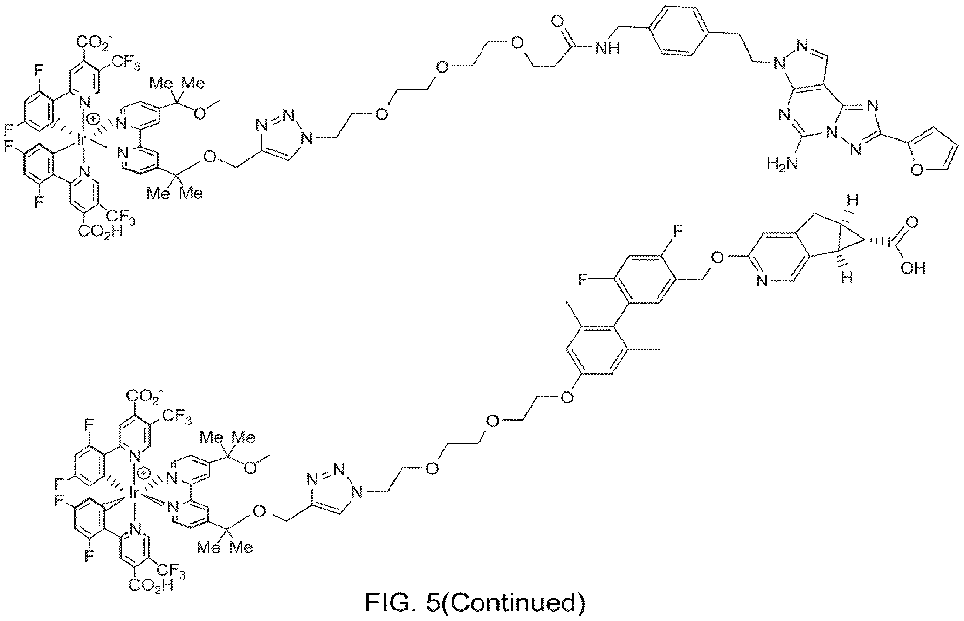

The biomolecular binding agent can be bonded to the catalyst. In some embodiments, the catalyst comprises a reactive handle or functionality for coupling the biomolecular binding agent. In some embodiments, for example, a catalyst can comprise one or more click chemistry moieties including, but not limited to, BCN, DBCO, TCO, tetrazine, alkyne, and azide. FIG. 4 illustrates various transition metal photocatalysts of Formula (I) having a reactive functionality for coupling a biomolecular binding agent. As illustrated in FIG. 4, an alkylene oxide linker of varying length can be employed between the reactive functionality and the coordinating ligand. Length of the alkylene oxide linker, such as ethylene oxide, can be chosen according to several considerations, including steric condition of the target site. FIG. 5 illustrates several transition metal photocatalysts of Formula (I) forming a conjugate with a biomolecular binding agent according to some embodiments.

### III. Systems for Proximity Based Labeling

In another aspect, systems for proximity-based labeling are described herein. A system, for example, comprises a conjugate including a catalyst coupled to a biomolecular binding agent, and a protein labeling agent activated by the catalyst for binding to a protein. The conjugate can comprise any catalyst and biomolecular binding agent described herein, including the embodiments detailed in Section II above. The catalyst, for example, can have electronic structure to activate the protein labeling agent to a reactive intermediate via energy transfer. Moreover, the protein labeling agent can comprise any of the labeling agents described herein, including the protein labeling agents set forth in Section I above. Specific identity of the conjugate and associated protein labeling agent can be selected according to several considerations, such as the chemical nature and/or steric requirements of the biological environment to be mapped with the proximity-based labeling system.

Systems for proximity-based labeling described herein can be employed in various applications. In some embodiments, the systems enable target identification, wherein the conjugate and associated protein labeling agent permit identification of one or more molecules in a biological context by proteomics. Additionally, systems comprising the conjugate and protein labeling agent facilitate interactome mapping. Targeting a conjugate and protein labeling agent allows detection and identification of one or more molecules and neighboring interactors in a biological context by proteomics. Identification of such molecules by systems described herein can permit enrichment and/or purification of such molecules and neighboring interactors. Additionally, systems comprising a conjugate and protein labeling agent further enable detection and identification of one or more molecules in a biological context via microscopy.

### IV. Methods of Proximity-Based Labeling

In another aspect, methods of proximity-based labeling are described herein. A method of proximity-based labeling comprises providing a conjugate comprising a catalyst coupled to a biomolecular binding agent, activating a protein labeling agent to a reactive intermediate with the catalyst, and coupling the reactive intermediate to a protein. The conjugate can comprise any catalyst and biomolecular binding agent described herein, including the embodiments detailed in Section II above. Moreover, the protein labeling agent can comprise any of the labeling agents described herein, including the protein labeling agents set forth in Section I above. Specific identity of the conjugate and associated protein labeling agent can be selected according to several considerations, such as the chemical nature and/or steric requirements of the biological environment to be mapped with the proximity-based labeling system. In some embodiments of proximity-based labeling, the catalyst can be provided in the absence of a biomolecular binding agent.

Methods described herein can be employed to map various biological environments, including local areas of cellular membranes and/or the local extracellular environment. The conjugate comprising the catalyst and biomolecular binding agent may be targeted to a specific local region of a cellular membrane, such as a receptor of interest. Activation of the protein labeling agent can identify protein(s) and/or other molecules in the targeted local region. Notably, the activated protein labeling agent can also identify or label molecules associated with another cell in contact with the targeted cellular region. Therefore, intercellular interactions and intercellular environments can be elucidated and mapped with systems and methods described herein. The foregoing methods enable interactome mapping, and the identification of one or more molecules and neighboring interactors in a biological context by proteomics. Identification of such molecules by methods described herein can permit enrichment and/or purification of such molecules and neighboring interactors.

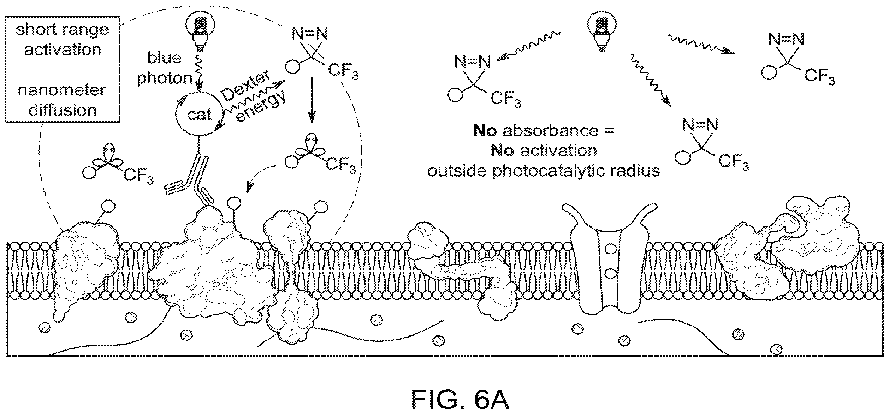

FIG. 6A illustrates operation of proximity-based labeling systems and methods described herein according to some embodiments. As illustrated in FIG. 6A, a conjugate comprising a photocatalyst and antibody targets a specific local area of the cellular membrane. The photocatalyst is irradiated to produce the excited state followed by energy transfer to the diazirine protein labeling agent. The diazirine protein labeling agent comprises a tag for identification purposes in the analysis. The energy transfer activates the protein labeling agent to the reactive carbene. The reactive carbene binds to proteins and/or other biomolecules within the tight diffusion radius, as described herein. Diazirine labeling agent outside the activation or energy transfer radius of the photocatalyst is not activated. Additionally, proteins and/or other biomolecules outside the diffusion radius of the reactive carbene are not labeled. In this way, high resolution proximity-based labeling can be achieved.

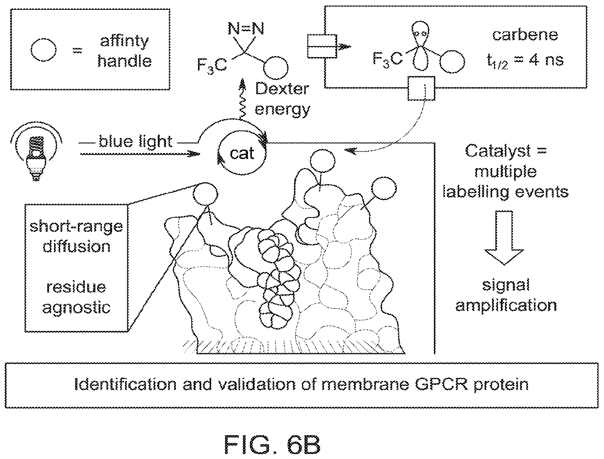

FIG. 6B illustrates operation of proximity-based labeling systems and methods described herein for identification and validation of membrane G protein-coupled receptors (GPCR) according to some embodiments. As provided in FIG. 6B, a conjugate comprises a photocatalyst coupled to a biomolecular binding agent having specificity for the GPCR. The conjugate binds to a local site of the GPCR. The photocatalyst is irradiated with blue light to provide an excited state followed by energy transfer to the diazirine protein labeling agent. The diazirine protein labeling agent comprises an affinity handle or tag for identification purposes in the analysis. The energy transfer activates the protein labeling agent to the reactive carbene. The reactive carbene binds to proteins and/or other biomolecules within the tight diffusion radius, as described herein. Multiple labeling events may occur at the local GPCR site leading to signal amplification.

These and other embodiments are further illustrated by the following non-limiting examples.

### EXAMPLE 1 - Photocatalytic diazirine sensitization for protein labeling

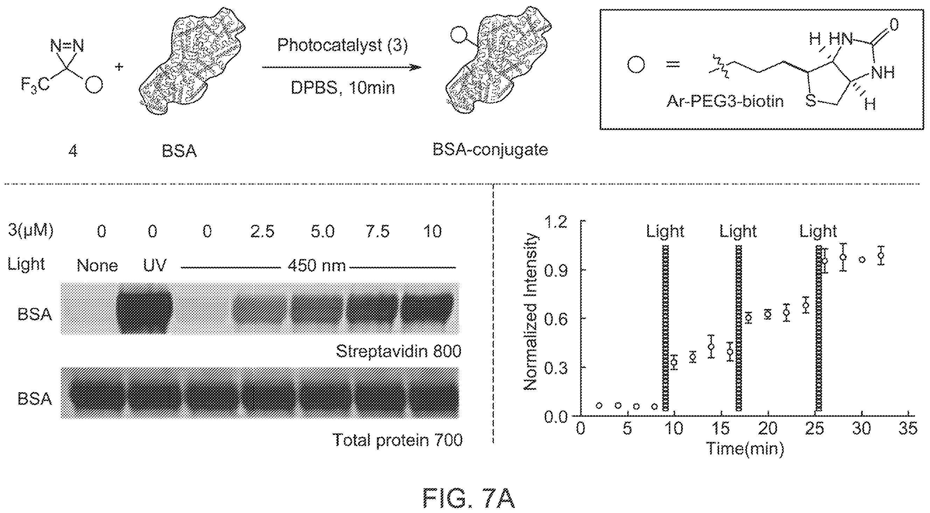

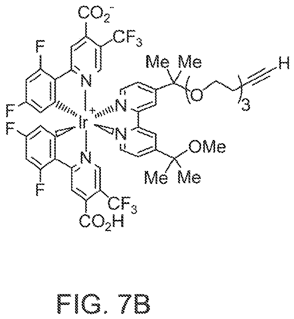

It was demonstrated that carbenes generated through photocatalytic diazirine sensitization could label proteins. As illustrated in FIG. 7A, photocatalyst **3** was combined with diazirine **4** and BSA in DPBS to afford reaction mixtures with 100 µL total solution volume and desired component concentrations. Photocatalyst **3** is illustrated in FIG. 7B, and Table I summarizes the sample conditions. These samples were then either placed in the dark, irradiated with UV (375 nm) light, or irradiated with visible (450 nm) light in a biophotoreactor for 10 minutes at 100% intensity. 30 µL samples were then removed, combined with 10 µL of 4x reducing Laemmli sample buffer (5% β-mercaptoethanol), vortexed, and heated at 95 °C for 10 minutes. 10 µL of each sample was then analyzed by Western blot.

**Table I - Sample Conditions**

| Sample | Diazirine **4** (µM) | Photocatalyst **3** (µm) | BSA (µM) | Light |
|---|---|---|---|---|
| 1 | 100 | 0.0 | 10.0 | None |
| 2 | 100 | 0.0 | 10.0 | 375 nm |
| 3 | 100 | 0.0 | 10.0 | 450 nm |
| 4 | 100 | 2.5 | 10.0 | 450 nm |
| 5 | 100 | 5.0 | 10.0 | 450 nm |
| 6 | 100 | 7.5 | 10.0 | 450 nm |
| 7 | 100 | 10.0 | 10.0 | 450 nm |

Biotinylation of BSA was detected for Sample 2. When a solution of BSA and biotinylated diazirine probe was irradiated with 450 nm light (Sample 3), the degree of biotinylation was less than 0.5%, establishing that the diazirine presents minimal background signal at this wavelength. However, in the presence of water soluble photocatalyst **3,** catalyst dependent biotinylation of BSA was observed. Photocatalytic labeling of BSA was further confirmed through intact protein mass spectrometry. Unlike prior enzyme-based labeling methodologies, this approach requires continuous delivery of visible-light to sustain diazirine sensitization via the photocatalyst **3** for protein labeling. This feature was exploited to demonstrate how turning the light source on or off affords fine temporal control over the labeling process, as illustrated in FIG. 7A.

### EXAMPLE 2 - Antibodyphotocatalyst conjugate and proximity-based labeling using the same

A secondary antibody-photocatalyst conjugate was prepared as a general entry point for spatially targeted photocatalytic proximity labeling on cell surfaces. A goat anti-mouse (Gt/α-Ms) antibody was first decorated with azide groups through reaction with azidobutyric acid *N-*hydroxysuccinimide ester, and then conjugated to alkyne-bearing iridium catalyst via click chemistry, resulting in an antibody-photocatalyst ratio of 1:6. The iridium catalyst is the iridium complex **3** illustrated in FIG. 7B.

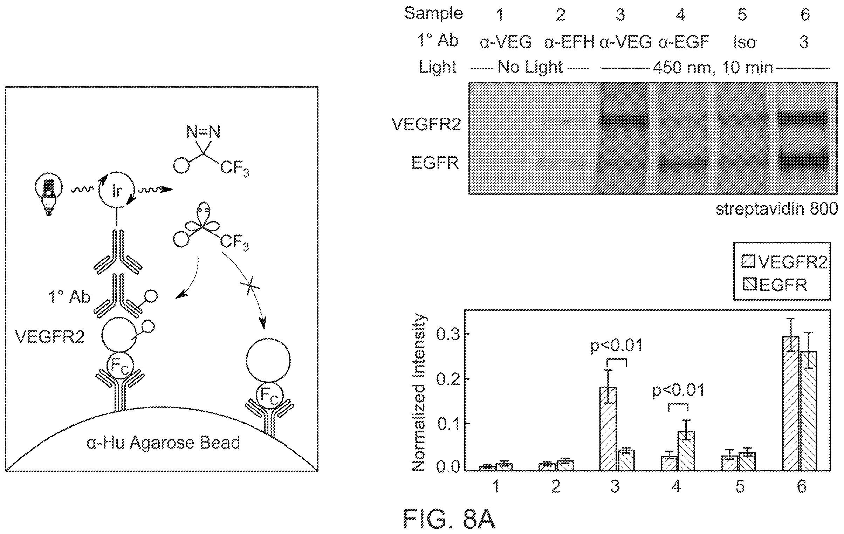

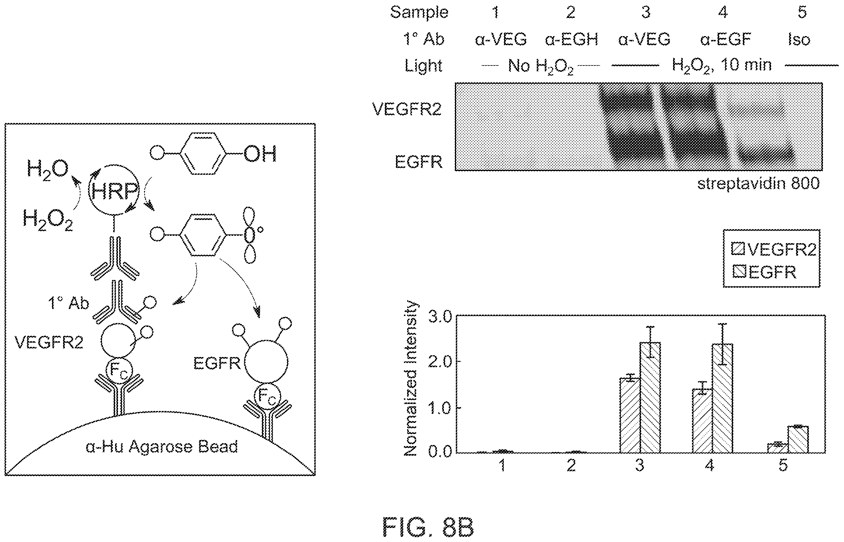

Next, to address protein targeted labeling on a surface, a model system was prepared containing human Fc-tagged vascular endothelial growth factor receptor 2 (VEGFR2) and epidermal growth factor receptor (EGFR) proteins attached to α-human immunoglobulin-G (IgG) agarose beads (FIG. 8A). These beads were sequentially incubated with 20 a Ms/α-VEGFR2 antibody and Ir-Gt/α-Ms to position the iridium catalyst close to the VEGFR2 proteins on the bead surface. Irradiation of these beads with 450 nm light in the presence of a diazirine-biotin probe afforded selective labeling of VEGFR2 over EGFR. When Ms/α-EGFR was used as the primary antibody, the selectivity of labeling was reversed. It is important to note that an analogous experiment, using peroxidase-based labeling, was incapable of differentiating between EGFR or VEGFR2, as illustrated in FIG. 8B.

### EXAMPLE 3 - Microevironmental Mapping on Cellular Membranes

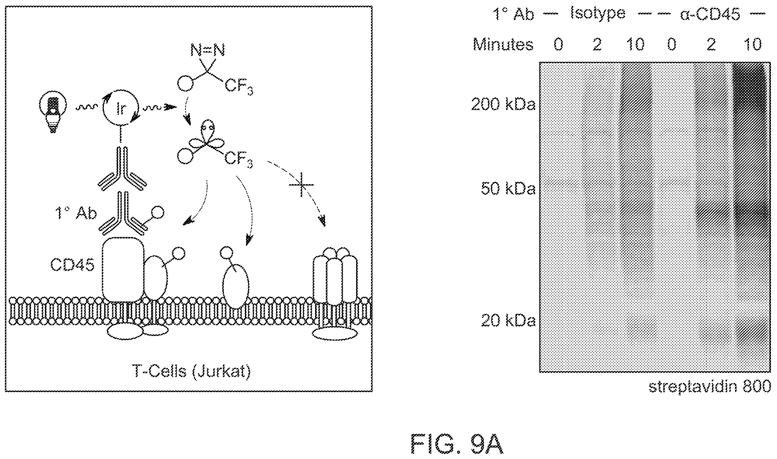

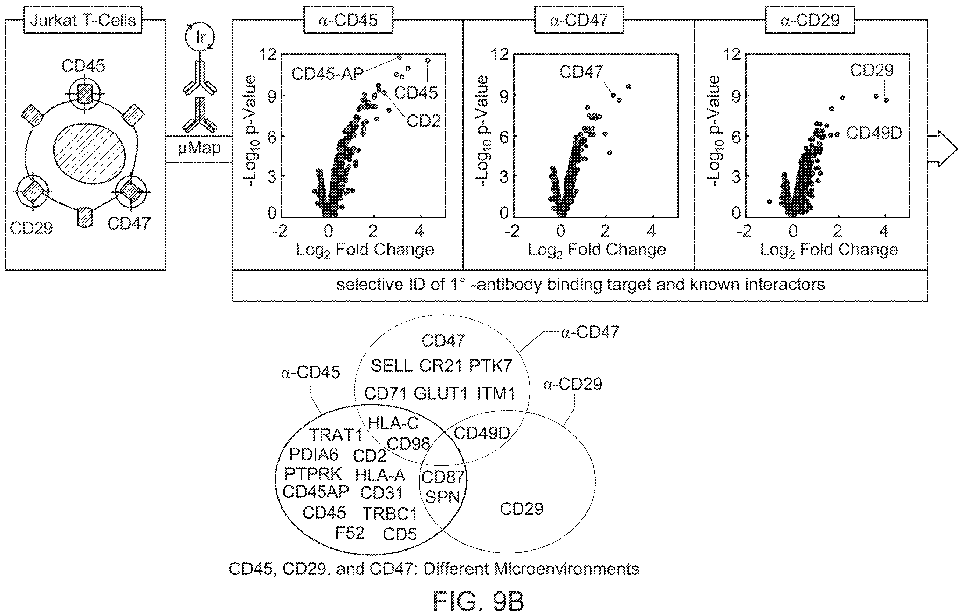

Antibody-targeted photocatalytic diazirine activation (i.e. µMap) was applied to the surface of live cells. For these experiments, addition of the antibody to the cell surface 5 was maintained at 4 °C to limit antibody-mediated protein crosslinking. CD45, a highly abundant tyrosine phosphatase on T cell surfaces involved in antigen receptor signaling was selected as an initial target. Western blot analysis of CD45-targeted µMap on Jurkat cells showed light and time dependent protein biotinylation compared to the isotype targeting control (FIG. 9A). Next, tandem mass tag (TMT)-based quantitative proteomic analysis of streptavidin enriched proteins was used to identify CD45 and two known associators (CD45AP and CD2) as part of a wider subset of enriched cell membrane proteins (FIG. 9B).

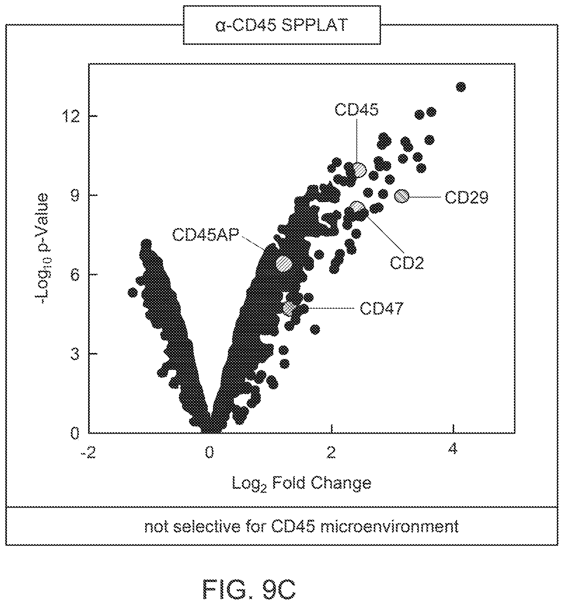

With proof of concept for cell surface labeling in hand, it was explored whether µMap could differentiate between spatially separated microenvironments on the same cell membrane. To this end, CD29 and CD47 were selected as ideal targets with no known co-spatial association on the cell surface. Indeed, µMapping of CD45, CD29, or CD47 on Jurkat cells resulted in the enrichment of unique sets of proteins, which included both known (CD29:CD49D, CD45:CD45AP:CD2) and previously unknown interactors (Fig. 9B). Crucially, although several proteins were shared between pairs of targeted proteins, none were shared across all three, validating the ability of µMap to discriminate between unrelated microenvironments. In contrast, when employing state-of-the-art peroxidase-based proximity labeling methods, cell surface CD45 and associated proteins were not selectively resolved from CD29 or CD47 (FIG. 9C).

### EXAMPLE 4 - Microevironmental Mapping on Cellular Membranes

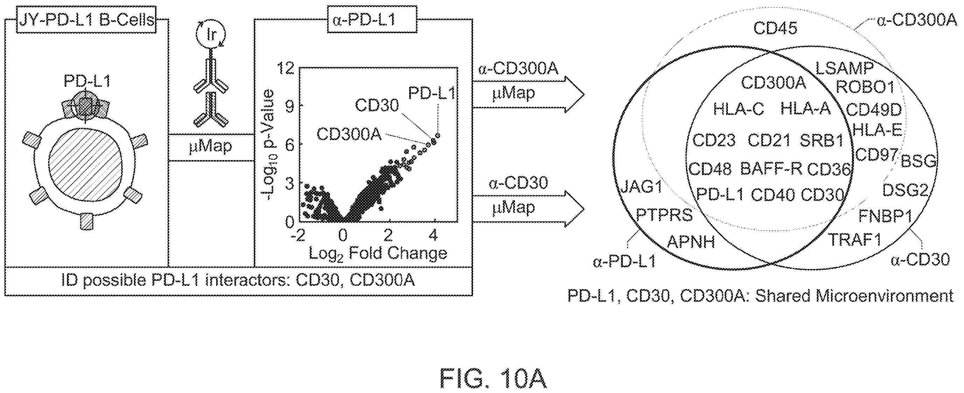

The selectivity of compositions and methods described herein was also harnessed to investigate the proximal protein interactome of PD-L1 in B cells. As has been well established, PD-L1 plays an important role in cancer cells as an immune checkpoint ligand that can accelerate tumor progression via suppression of T cell activity. In the event, PD-L1 targeted µMap revealed CD30, a member of the tumor necrosis factor receptor family, and CD300A, an immune inhibitory receptor 5 (FIG. 10A), as potentially new interactors based upon significant enrichment. These results highlight the potential of µMapping to provide new insights with respect to the microenvironments of checkpoint proteins.

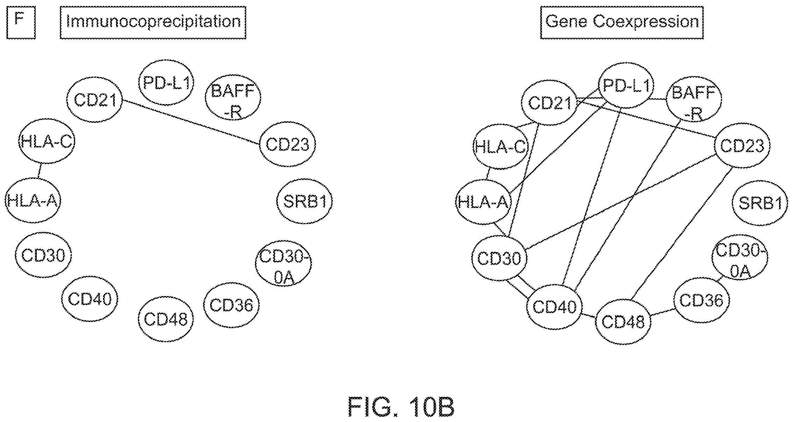

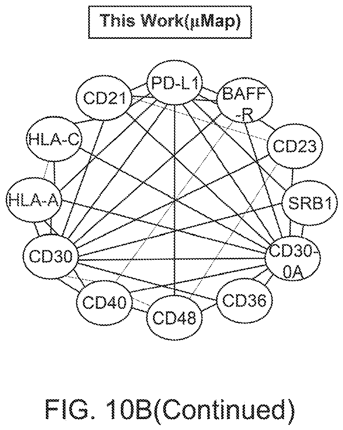

To further validate the enriched subset of proteins identified by PD-L1 µMapping, 10 targeted labeling of these two highly enriched proteins were performed. Targeted µMapping of these proteins within the PD-L1 microenvironment should, therefore, afford similar enrichment lists, verifying their spatial association. Indeed, it was found that α-CD30, α-CD300A, and α-PD-L1 directed µMapping identified the same set of 12 surface receptors (FIG. 10B).

### EXAMPLE 5 - Intra/Extrasynaptic µ-Mapping within a two-cell system

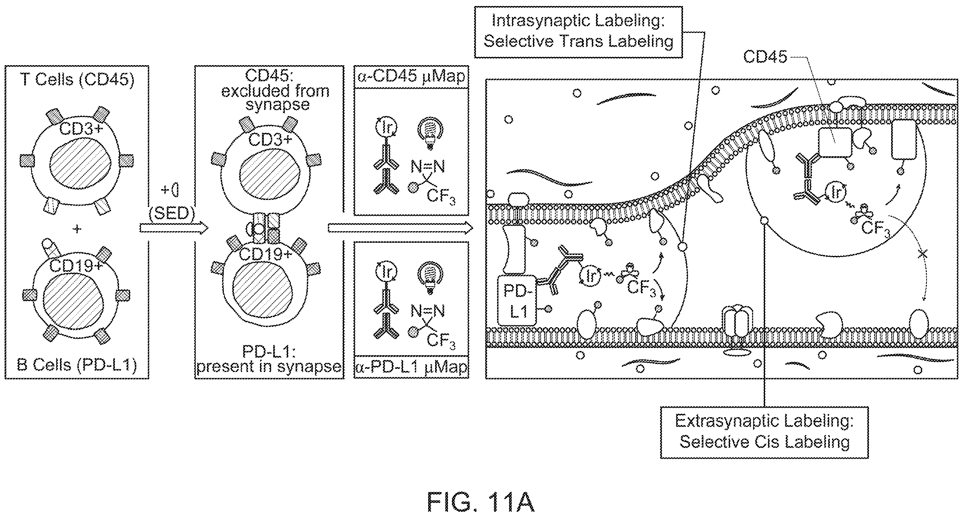

It is well recognized that the development of new therapeutic oncology strategies will require an understanding of the underlying mechanisms of intercellular communication, particularly within the context of T-cell activation and differentiation. Furthermore, given that localization of PD-L1 is found within the T cell/antigen presenting cell (APC) immunosynapse (i.e. at the interface between two immunointeractive cells), it was hypothesized that PD-L1 directed µMapping with conjugates and methods described herein should lead to not only biotinylation of a PD-L1 expressing APC surface (cis-labeling), but also to the biotinylation of the adjacent synaptic T cell (*trans*-labeling) (FIG. 11A). As an important control experiment, it was further posited that when targeting a protein excluded from the synapse, such as CD45RO, the diffusion minimized radius of µMap would preclude biotinylation of the distant *trans-cell* membrane.

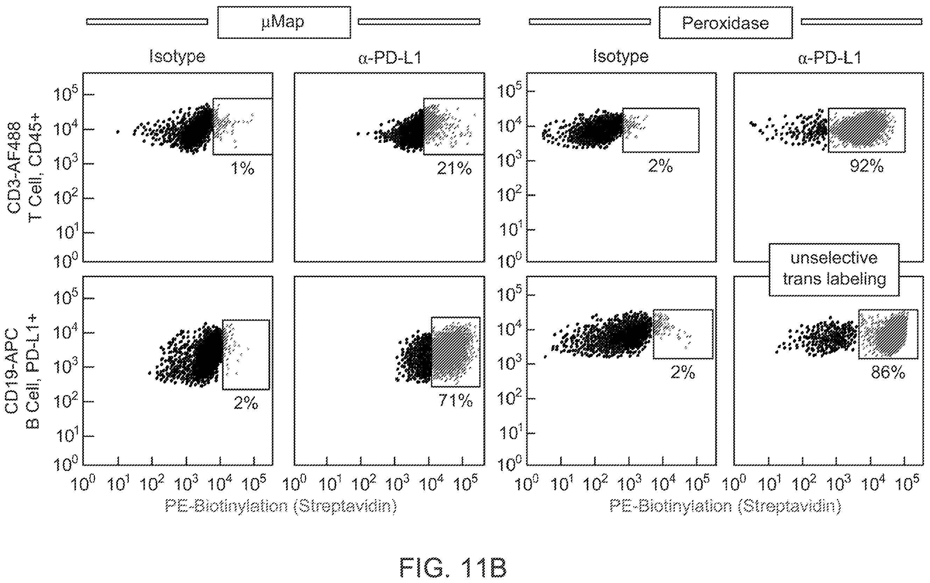

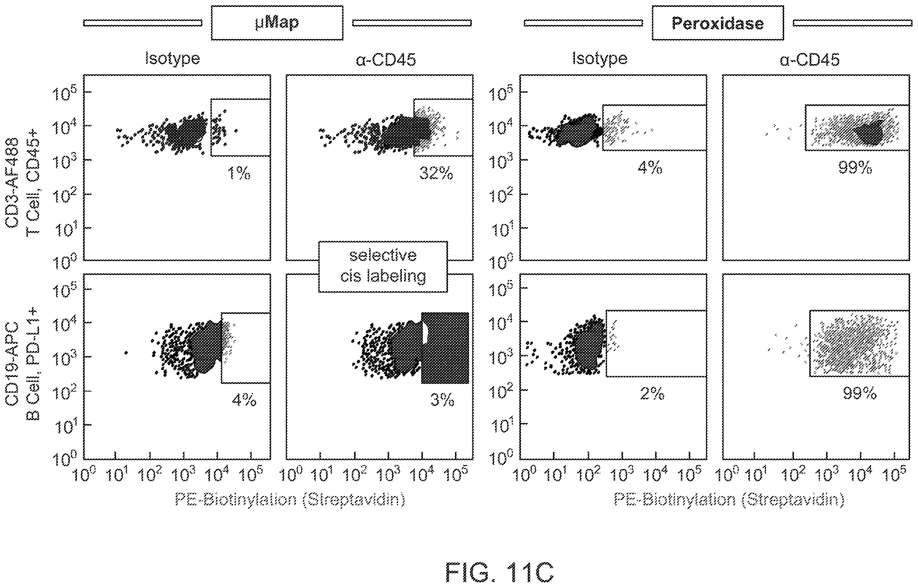

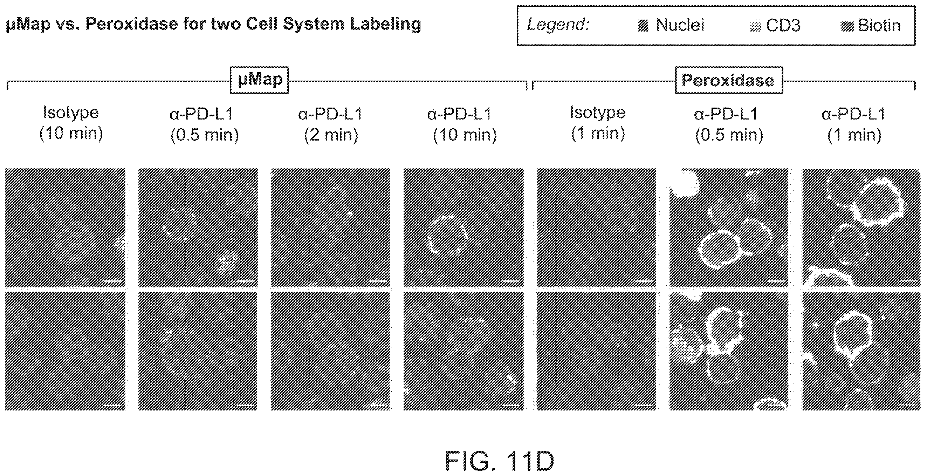

PD-L1- and CD45-targeted µMapping was evaluated in a two-cell system composed of PD-L1-expressing JY- B-lymphocytes as the antigen presenting cell and Jurkat T-lymphocytes uniquely expressing PD-1 and the CD45RO isoform. Given that immune cell-APC interactions are driven by the binding of multiple receptor types (e.g. adhesion, co-stimulatory/co-repressor, and T cell receptor (TCR)-major histocompatibility complex (MHC) 5, staphylococcal enterotoxin D (SED) was employed to facilitate MHC class II and TCR engagement and promote B cell / T cell immune synapse formation and signaling (FIG. 11A). After the application of the blue light irradiation-based µMap protocol, the extent of *cis*/*trans* labeling selectivity was assayed via flow cytometry analysis. As anticipated, PD-L1-targeted µMap resulted in both *cis-* and *trans*-cellular labeling, while CD45RO-targeted µMap led to selective *cis*-labeling on the CD45RO-expressing Jurkat cells (Figs. 11B and 11C) without any labeling of the adjacent B-cell. In stark contrast to µMap, peroxidase-based proximity labeling of PD-L1 or CD45RO within this two-cell system led to complete labeling of both cell types within 30 seconds, clearly visualized through flow cytometry and confocal microscopy (FIGS. 11B, 11C and 11D). In comparison, PD-L1 targeted µMap showed high selectivity for *trans*-labeling solely at the *cis-* and *trans*-cellular contact regions (FIG. 11D). Importantly, applying the µMap technology on PD-1 in the Jurkat-JY co-culture system resulted in the reciprocal trend of *cis-* and *trans*-cellular labeling. Collectively, these findings clearly demonstrate that the capacity of µMap to elucidate protein-protein interactions can be directly translated towards the highly selective labeling of dynamic interfaces within complex multicellular systems.

### EXAMPLE 6 - Antibodyphotocatalyst conjugate and proximity-based labeling using the same

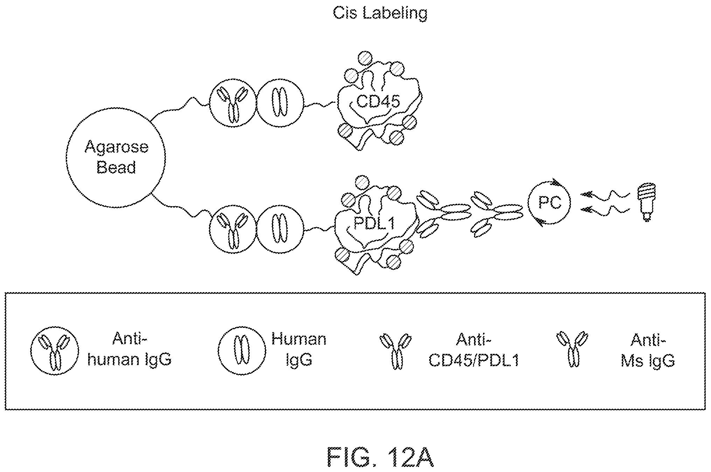

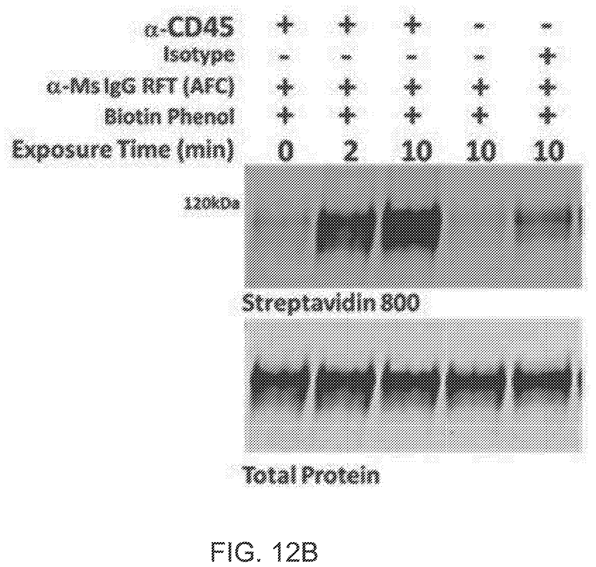

Conjugates comprising flavin photocatalyst in conjunction with phenol-based protein labeling agents can be employed in proximity-based labeling compositions and methods described herein. FIG. 12A illustrates bead-based protein labeling wherein CD45-Fc or PDL1-Fc fusion proteins are bound on the same beads followed by attached of a primary antibody and secondary antibody flavin conjugate (AFC). CD45 or PDL1 are then labeled in the presence of biotin phenol and visible light. FIG. 12B is Western blot analysis of light dependent CD45 biotinylation for the indicated time points.

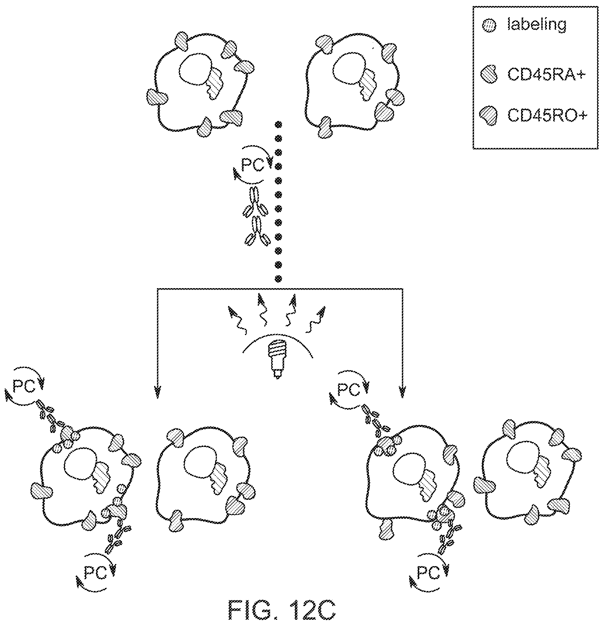

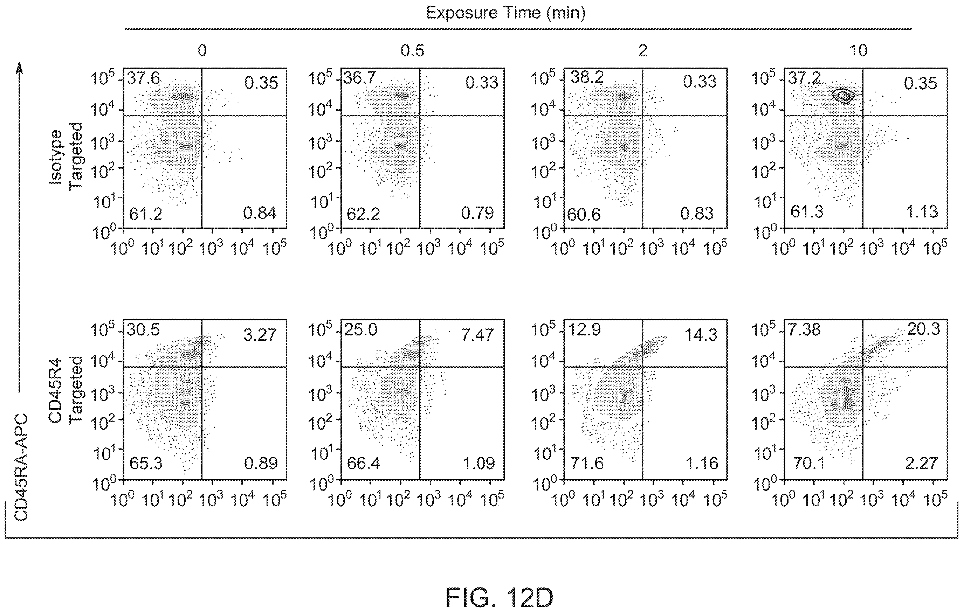

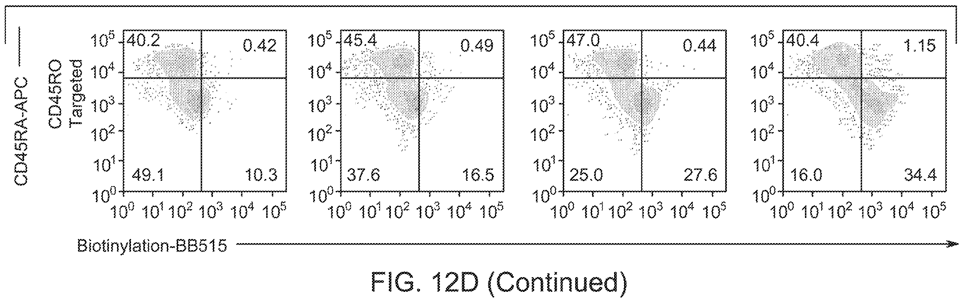

FIG. 12C is a schematic of CD45RO- or CD45RA-targeted cell labeling with AFC in mixed T cell populations. FIG. 12D is flow cytometry analysis of the photo labeling time course of CD45RA+ or CD45RO+ T cell biotinylation with Isotype (top), α-CD45RA + AFC (middle), and α-CD45RO + AFC (bottom)

### EXAMPLE 7 - - Microevironmental Mapping on Cellular Membranes

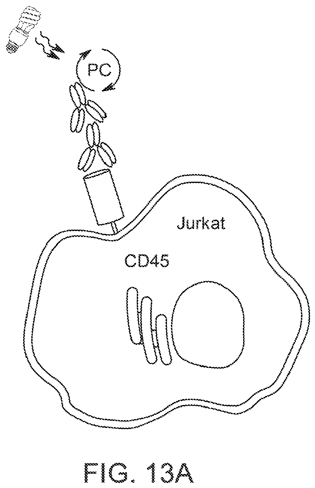

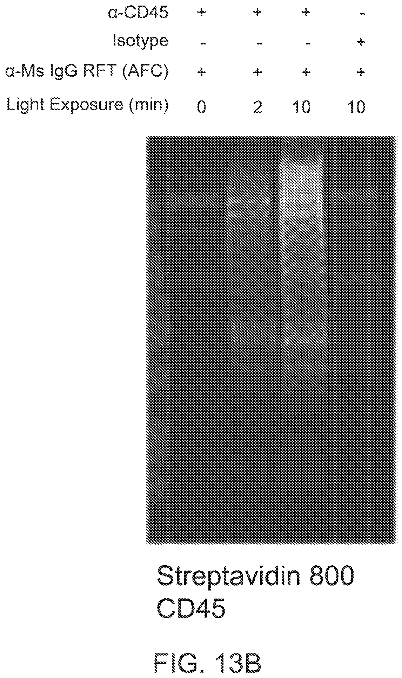

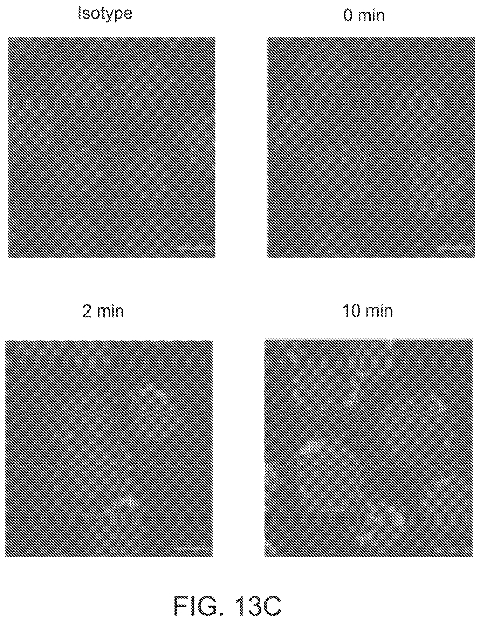

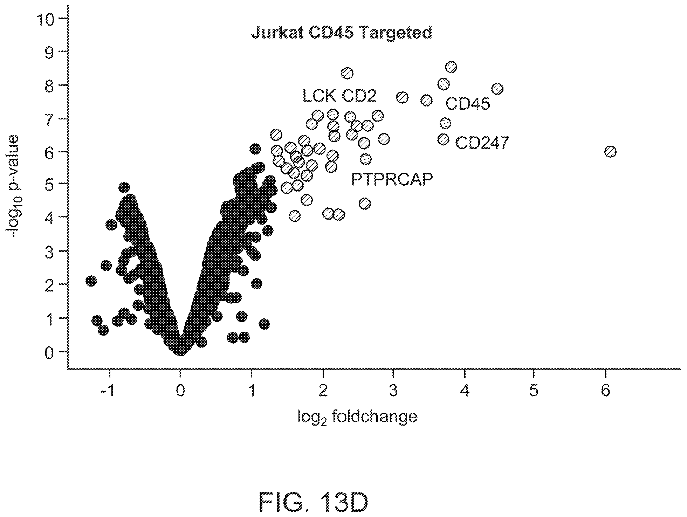

FIG. 13A is a schematic depicting photoproximity labeling of CD45 on Jurkat cells with secondary antibody flavin conjugate (AFC). FIG. 13B is Western blot analysis of CD45-targeted labeling of the Jurkat cells. Biotinylation levels increase as a function of duration of visible light irradiation with CD45-directed labeling, but not with isotype controls. FIG. 13C is confocal imaging of cells with CD45-directed labeling indicates that biotinylation (magenta stain) is both confined to the cell surface and light exposure time dependent. Nuclei are labeled with Hoechst stain and scale bars indicate 5 mm. FIG. 13D provides volcano plots of significance vs. fold-enrichment for targeted- vs isotype-targeted biotinylation of CD45 in Jurkat cells, following 2 min of blue light illumination, harvesting, capture on streptavidin beads and quantitative mass spectrometry-based proteomic analyses. Significantly enriched proteins (those with an FDR-corrected p-value < 0.05 and displaying a fold-enrichment above the isotype targeted control of > 2.5 (1.32 log₂-fold change)) are indicated as blue dots, known CD45 associators within this enriched group are indicated with orange dots and CD45 is indicated with a red dot (n=3 experiments).

### EXAMPLE 8 - - Microevironmental Mapping on Cellular Membranes

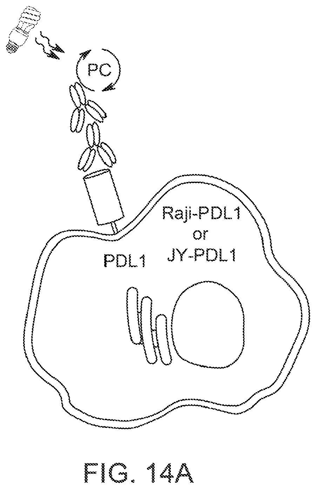

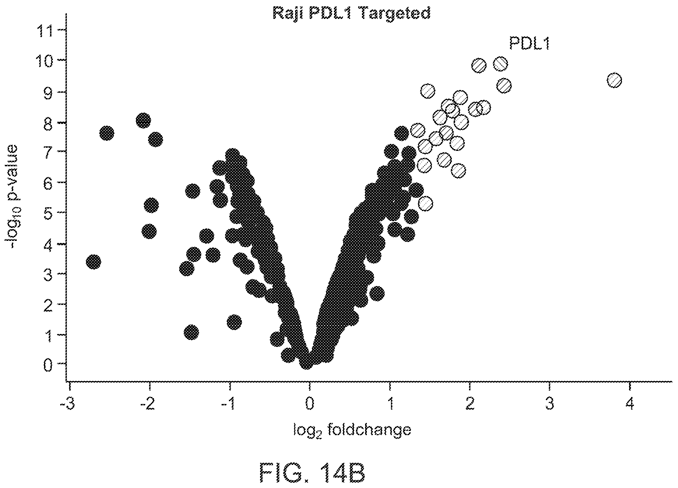

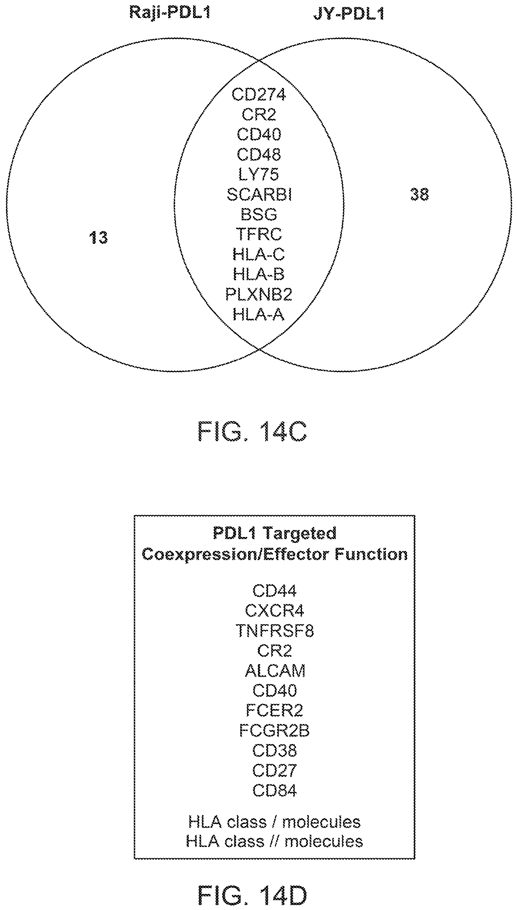

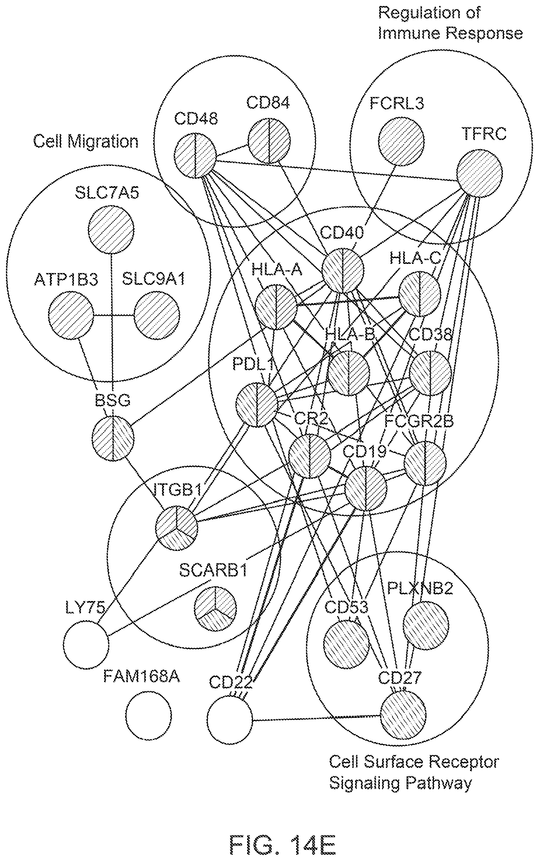

FIG. 14A is a schematic depicting photoproximity labeling of PDL1 on JY-PDL1 cells with secondary antibody flavin conjugate (AFC). FIG. 14B provides volcano plots of significance vs. fold-enrichment for targeted- vs isotype-targeted biotinylation of PDL1 on Raji cells expressing PDL1, following 2 min of blue light illumination, harvesting, capture on streptavidin beads and quantitative mass spectrometry-based proteomic analyses. Significantly enriched proteins (those with an FDR-corrected p-value < 0.05 and displaying a fold-enrichment above the isotype targeted control of > 2.5 (1.32 log₂-fold change)) are indicated as orange or blue dots, and PDL1 is indicated with a red dot (n=3 experiments). FIG. 14C is a Venn diagram of significantly enriched proteins identified from PDL1 targeted labeling on JY and Raji cells expressing PDL1. Proteins identified on both cell types are shown in the center. FIG. 14D is a list of significantly enriched proteins identified from PDL1 targeted on JY and Raji cells with known PDL1 related function. FIG. 14E is a string protein interaction network and GO term analysis of significantly enriched proteins for PDL1-targeted experiments. Shading indicates membership within broad gene ontology (biological process) terms. Nodes with multiple shaded tones indicate membership in more than one term. Thick edges indicate experimental evidence of interaction from StringDB while thin edges indicate interactions from other sources.

### EXAMPLE 9 - Intra/Extrasynaptic µ-Mapping within a two-cell system

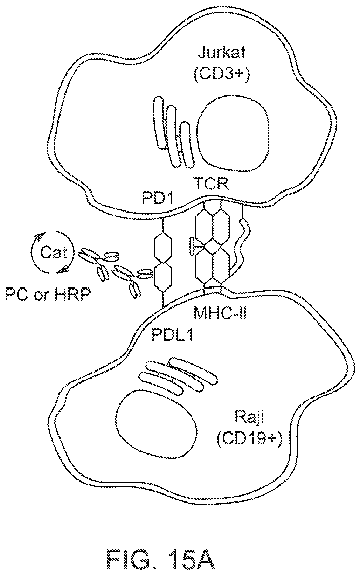

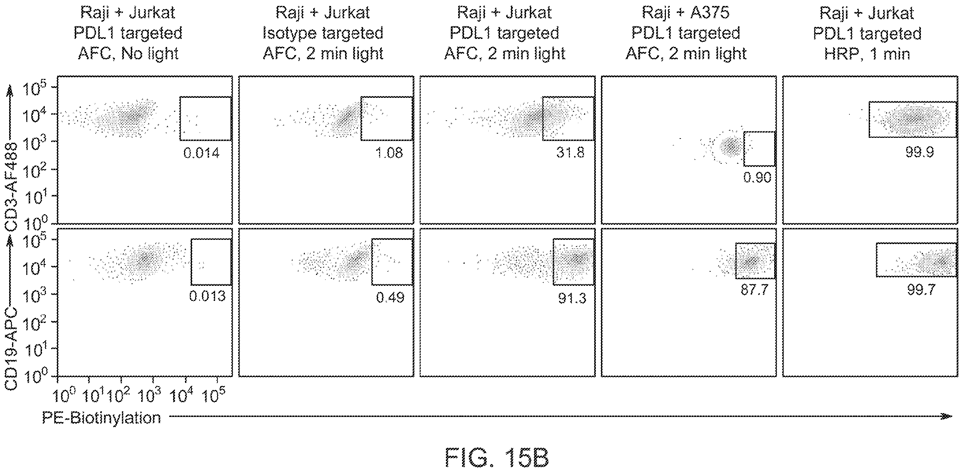

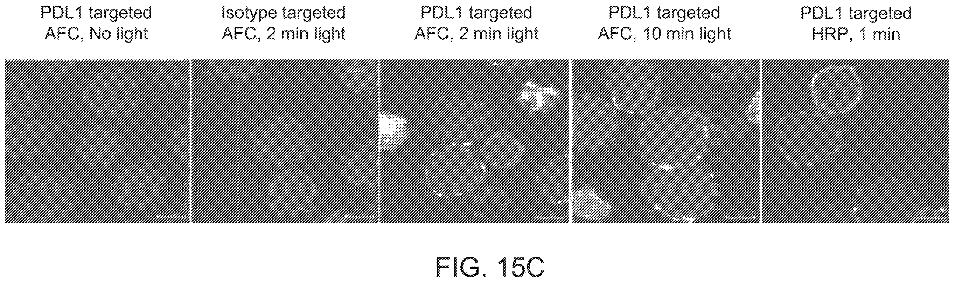

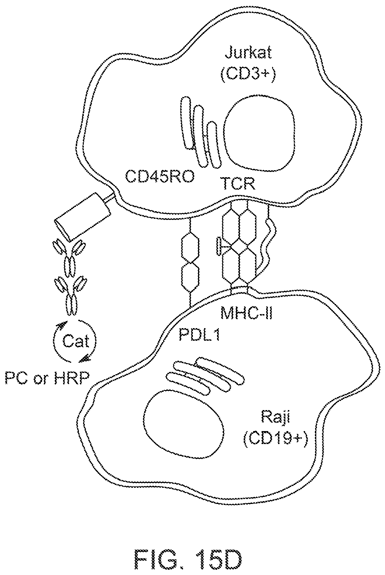

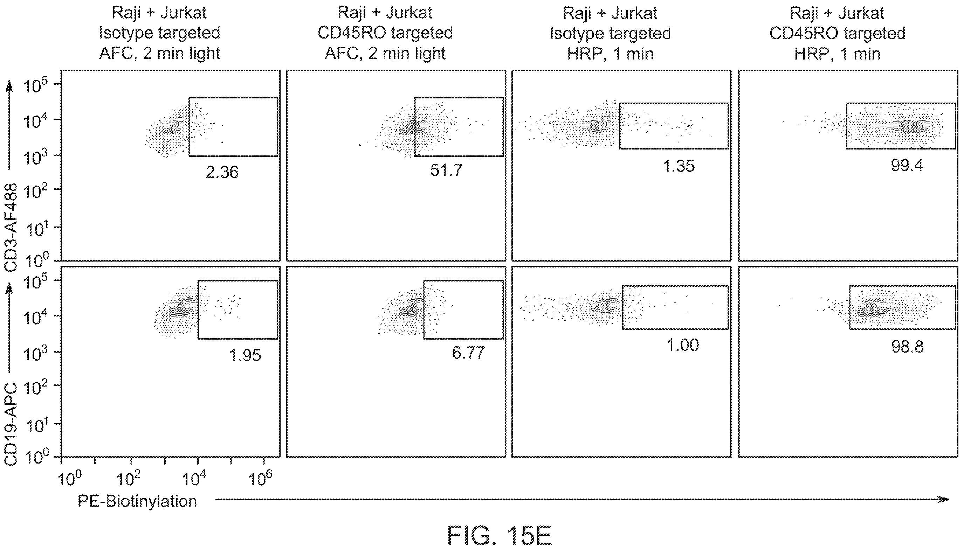

FIG. 15A is a schematic depicting a two-cell system consisting of engineered Jurkat and Raji cells. Antibody-targeted labeling with a photocatalyst (PC) or peroxidase (HRP) on PDL1 is illustrated. FIG. 15B is flow cytometry analysis wherein biotinylation is detected on both Raji and Jurkat cells with PDL1 targeting using the antibody flavin conjugate (AFC), but not Isotype targeting or in the absence of visible light irradiation. Transcellular labeling was not observed between PDL1-labeled Raji cells and suspended A375 cells. FIG. 15C is confocal microscopy imaging of the Raji-Jurkat two cell system with PDL1 targeting on Raji cells revealing labeling on both Raji cells and points of cellular contact on Jurkat cells using the AFC while excessive labeling on both cell types was observed with HRP (indicated with white arrows). Cells were imaged for biotinylation, CD3 surface expression, and nuclei. FIG. 15D is a schematic depicting a two-cell system consisting of engineered Jurkat and Raji cells. Antibody-targeted labeling with a photocatalyst (PC) or peroxidase (HRP) on CD45 is illustrated. FIG. 15E is flow cytometry analysis wherein targeting labeling of CD45RO on Jurkat cells (known to be excluded from the synapse) resulted in low levels of Raji transcellular labeling using an AFC and nearly quantitative labeling when HRP was used.

### EXAMPLE 10 - Selective Protein Labeling with Small Molecule-based Conjugates

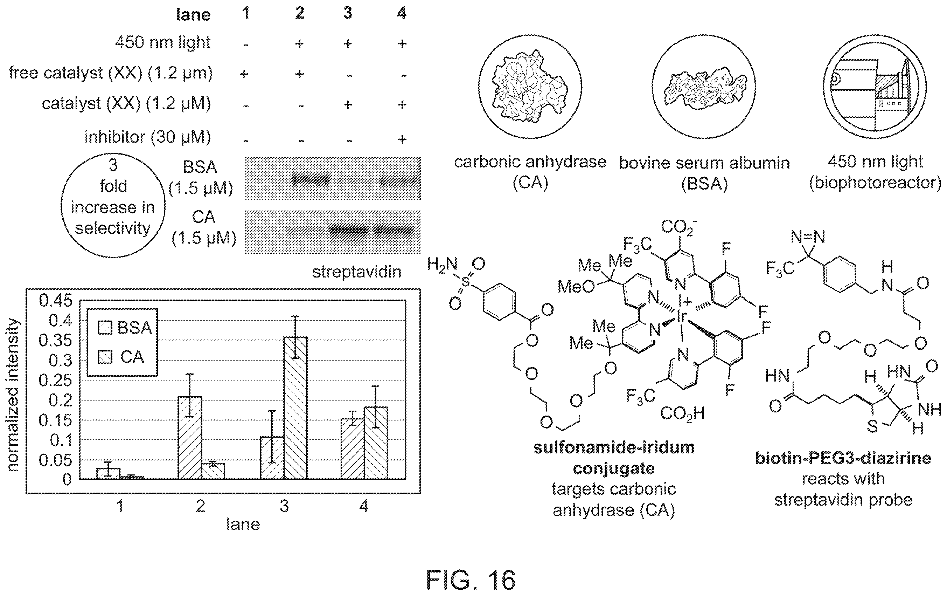

To first ascertain the capability of a small molecule-iridium conjugate to direct labeling towards a specific protein, a simple two-protein biochemical assay was designed. An equimolar ratio of the target protein, carbonic anhydrase (CA), with bovine serum albumin (BSA), as a competitor protein were chosen. Following this hypothesis, irradiation of the mixture in the presence of a biotin-tagged diazirine and sulfonamide-iridium conjugate would lead to selective labelling of CA over BSA. Analysis of labeling ratios by immunoblotting with streptavidin would provide an indication of both reaction efficiency and selectivity. Cognisant of the effect that the iridium catalyst may play on ligand binding, the catalyst-ligand conjugate was prepared with a PEG3 (triethylene glycol) linker to spatially separate the two components. Gratifyingly, after irradiation with 450 nm light for 10 minutes in the presence of biotin-peg3- diazirine, a 3.5:1 labeling in favor of the target protein CA was observed. FIG. 16 illustrates the experimental set up and results, including the sulfonamide-iridium conjugate and biotin-tagged diazirine.

In contrast, when labeling was performed in the presence of an unconjugated photocatalyst, BSA was selectively biotinylated over CA in a ratio of 5:1. Taken together, this comprises a 3-fold increase in labeling selectivity when using the Ir-ligand conjugate, providing confidence in this approach for targetID. Importantly, this selectivity was completely ablated when the targeted experiment was performed with an excess of the unconjugated sulfonamide ligand, confirming that the observed selectivity was the result of a ligand protein binding event. Moreover, significant enrichment of CA when performing the labelling in HEK293T cell lysate was found, validating the compatibility of this methodology with the most complex of biological settings.

### EXAMPLE 11 - Selective Protein Labeling with Small Molecule-based Conjugates

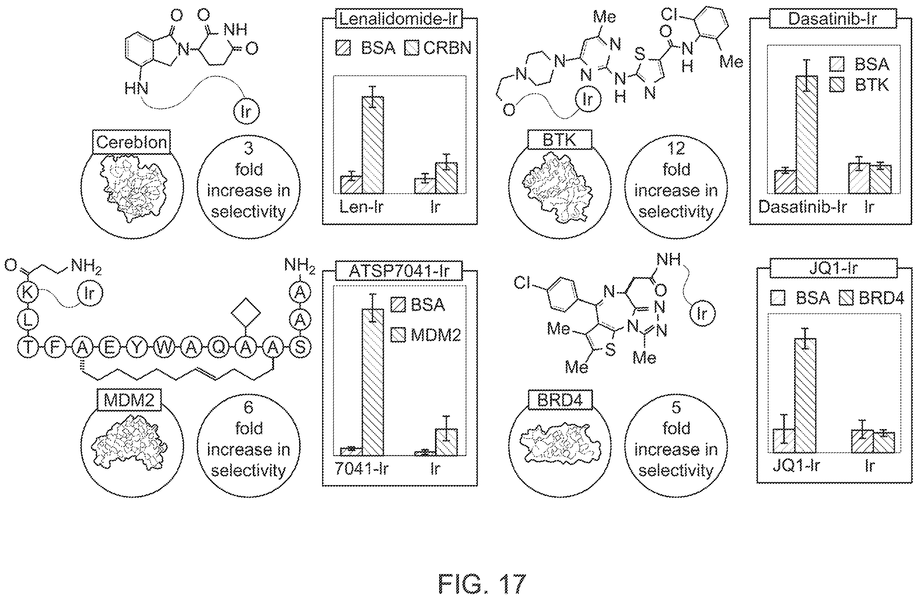

The generality of small-molecule based catalyst conjugates as a platform with regard to ligand-directed targeting identification of proteins was further investigated. Small molecule-iridium conjugates could be readily prepared in an operationally straight-forward manner using a coppercatalyzed azide-alkyne click reaction (CuAAC) between iridiumalkyne of FIG. 4 and a series of ligand-azide conjugates. The ability of the ligand-iridium conjugates to label their corresponding protein targets was determined by their selectivity versus BSA as illustrated in FIG. 17. Dasatinib, a marketed treatment for myelogenous leukemia displays nanomolar binding to Bruton's tyrosine kinase (BTK, 5 nM) was first examined The corresponding dasatinib-Irconjugate led to a 12-fold increase in labeling in favor of BTK over BSA by western blot in comparison to the control lane. Bromodomain inhibitor JQ-114 (50 nM binding to BRD4) was similarly effective at directing labeling, providing a 6-fold increase in BRD4 labeling over BSA. A less-potent binder was next examined, the multiple myeloma treatment lenalidomide, which acts a molecular glue with the protein cereblon (CRBN). Although this ligand displays a lower binding affinity (178 nM), it proved equally effective at directing the photocatalytic targetID methodology described herein, leading to 3-fold change versus off-compete control. Taken together, these data suggest that the amplification of signal afforded by the catalytic nature of this labelling platform is able to overcome the traditional challenges of PAL using weaker- affinity ligands. Furthermore, it was found that the selectivity of labelling could be increased with prolonged irradiation time, further supporting our catalytic labelling hypothesis.

It was also sought to establish if other targeting modalities, in addition to small-molecules, would be compatible with catalytic labelling technology described herein. The α-helical stapled cyclic peptide ATSP7041, which targets the E3 ligase MDM2, could be readily conjugated to the photocatalyst through a modified azido-lysine. Significant enrichment (3-fold) of the target protein compared to the free photocatalyst control was observed, as provided in FIG. 17. Synthesis of the corresponding inactive cyclic peptide, bearing a D-phenylalanine, led to no selectivity for MDM2 labeling over BSA, once again confirming the interactions are substrate specific and not based on background affinity.

### EXAMPLE 12 - Investigating Small Molecule Protein-Protein Interactions (PPIs)

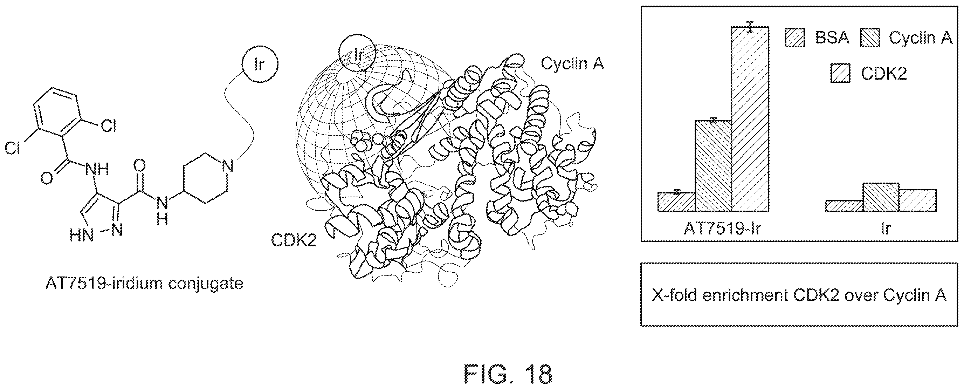

PPIs are essential to cell function and comprise a challenging class of targets for small molecule drug discovery. This challenge arises, in part, from the transient nature of these interactions which can make them difficult to detect biochemically. However, a number of prominent small molecule ligands are known to bind to protein complexes or to proteins that function through dynamic complexes. A method that could effectively distinguish the components of protein complexes would, therefore, prove an important tool for the investigation of PPIs. It was initially set out to study the small molecule AT7519, which binds to cyclin dependent kinase 2 (CDK2). It is well-established that CDK2 forms a PPI with the protein cyclin A, which contributes to the regulation of the cell cycle. As illustrated in FIG. 18, exposure of Ir-conjugated AT7519 to recombinant Cyclin A, CDK2, and BSA (as control) showed significant enrichment of both CDK2 (5-fold) and cyclin A (2-fold) over BSA control, exemplifying the ability of this methodology to capture transient interactions that would otherwise be challenging to examine.

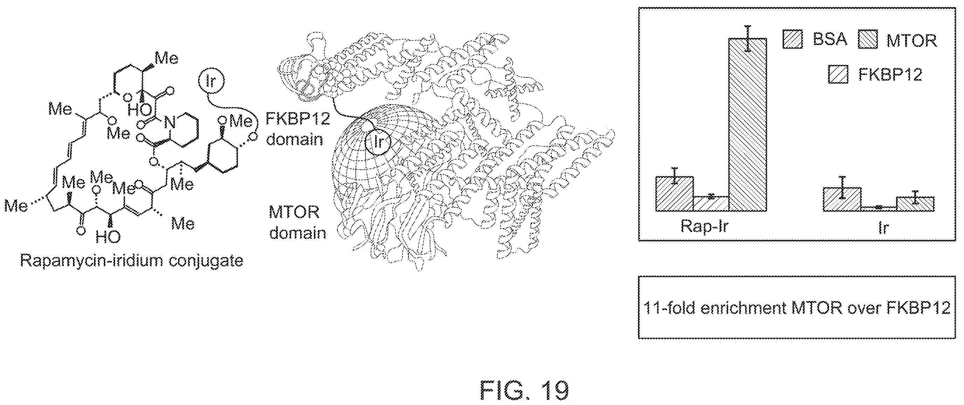

It was next set about exploring the rapamycin/mTOR axis in which rapamycin recruits FKBP12 to the mTOR complex, leading to suppression of the immune response. Notably, upon irradiation, both proteins were enriched compared to controls (FKBP12: 2-fold, mTOR: 12-fold), despite any disruption to binding efficacy caused by the pendant Ir-catalyst, as illustrated in FIG. 19.

### EXAMPLE 13 - Investigating Protein-Protein Interactions (PPIs)

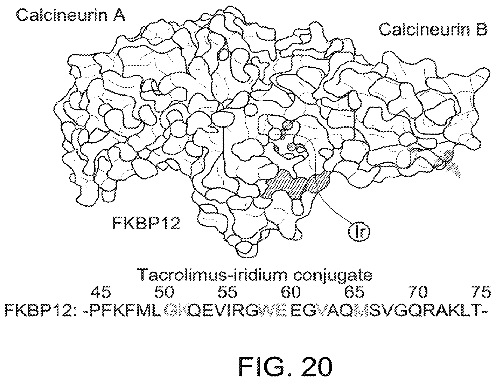

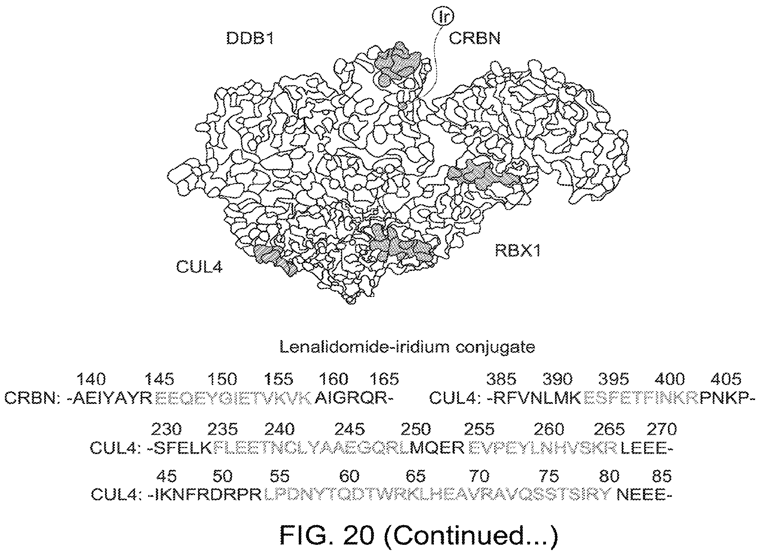

It was questioned whether the tight labeling of the µMap platform with conjugates and labeling agents described herein could be exploited to identify ligand binding sites through a combination of Western blotting and MS² analysis. To best explore this, the three-protein complex Calcineurin A/FKBP12/Calcineurin B was chosen, which binds to the macrolide tacrolimus. Upon synthesis of a range of tacrolimus-Ir conjugates with different linker lengths, it was found that short linkers led to labeling directly around the binding site of the complex, allowing footprinting of the molecular recognition site. However, with increasing tether length the labeling radius increased to include other members of the protein complex, changing the primary site of labeling. MS² analysis of the PEG3 linker supported this data, showing labeled residues directly adjacent to the binding site (FIG. 20, left).

A four-protein complex was then examined where the neighboring proteins do not directly interact with the small molecule ligand, to demonstrate that labeling of protein interactors through space can be achieved. For this, the E3 ligase complex CRBN/DDB1/Cul4a/RBX1 was chosen. In line with previous examples described herein, when a short tether was employed, only CRBN the primary target was labeled, however with increasing tether length, the neighboring proteins in the E3 ligase complex could be captured with this technology. Proteomic analysis of the labeling reaction employing a PEG3 linker showed a molecular footprint of the small molecule binding site on CRBN in addition to labeled sites on the neighboring Cul4a, that presumably arise from secondary protein-protein interactions (FIG 20, right).

### EXAMPLE 14 - Identification of Cell Surface Receptors

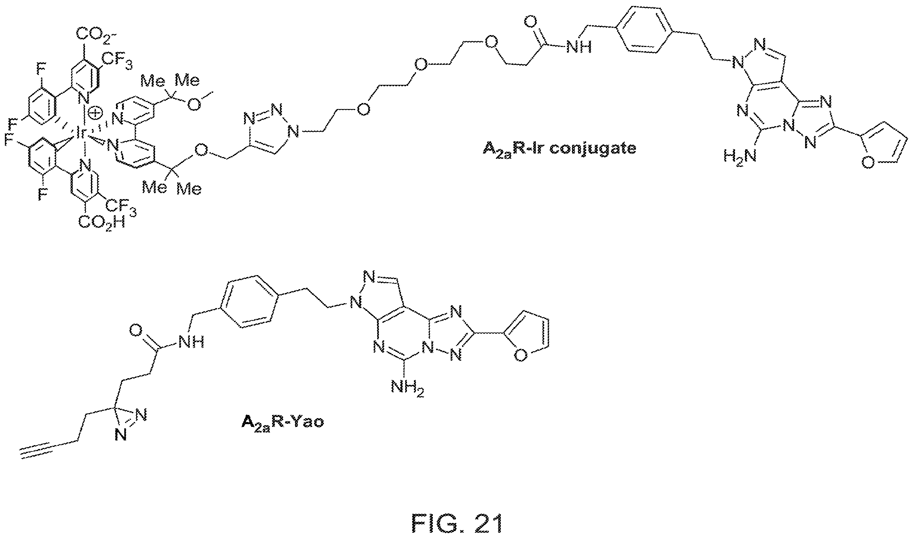

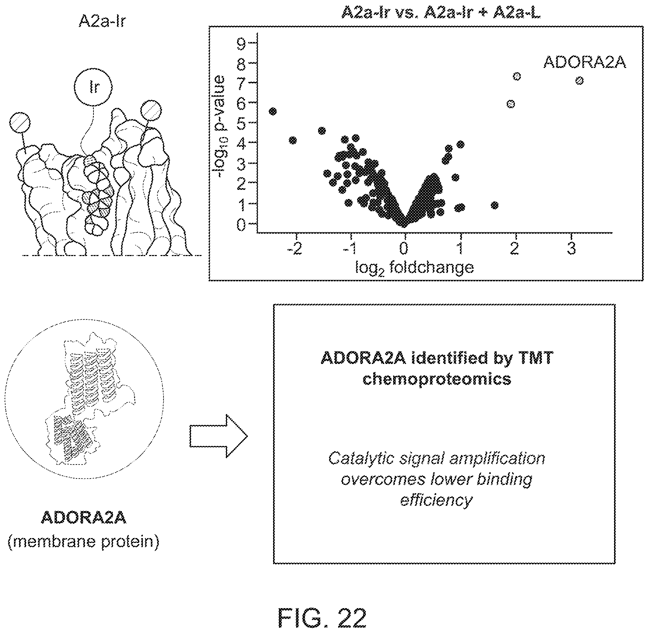

The adenosine receptor A2a (ADORA2A) as an exemplary membrane target. This GPCR has become an important target for immunotherapy, but critically, has never been identified through live cell chemoproteomics. Using a reported ligand for ADORA2A, **A2a,** an Irconjugate **(A2a-Ir)** was synthesized and a tethered diazirine-conjugate, as described by Yao **(A2a-Dz)** (FIG. 21). The binding affinities of the small molecule-conjugates were then measured to ensure retained potency for the proposed target upon conjugation. While the Yao diazirine retained binding close to that of the parent compound (0.8 nM vs 4.8 nM for the parent A2a), surprisingly, the Irconjugate displayed >100-fold lower binding affinity (643 nM). Nonetheless, the photocatalytic labelling methodology described herein on ADORA2A expressing HEK293T cells, followed by streptavidin immunoprecipitation and western blot analysis, revealed a stark difference in labelling between the stoichiometric Yao-type diazirine-A2a, and A2a-iridium probe. Immunostaining showed no signal corresponding to enriched ADORA2A following labelling with Yao-type diazirine-A2a; however, significant enrichment was observed using the photocatalytic-labelling platform described herein. TMT-based chemoproteomic analysis of these reactions confirmed the initial result, with photocatalytic-labeling method described herein showing a 3 log₂ fold change enrichment for ADORA2A, providing indisputable target identification of a GPCR. In contrast, using the stoichiometric diazirine, ADORA2A was not statistically enriched, in line with previous data. (FIG. 22). Crucially, the significant loss in affinity of the ligand-iridium conjugate to its target protein is far outweighed by the catalytic amplification in signal conferred by this labeling platform.

### EXAMPLE 15 - Identification of Cell Surface Receptors

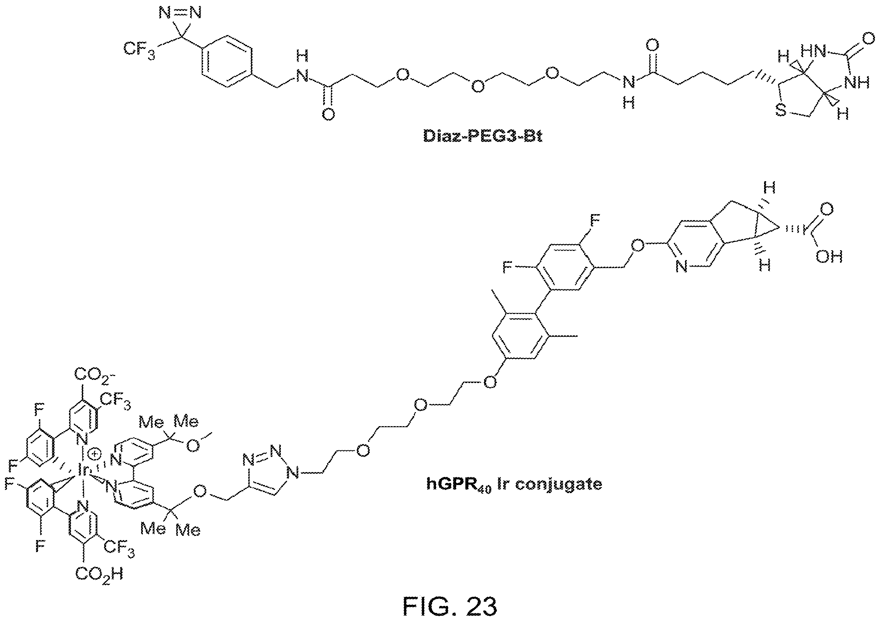

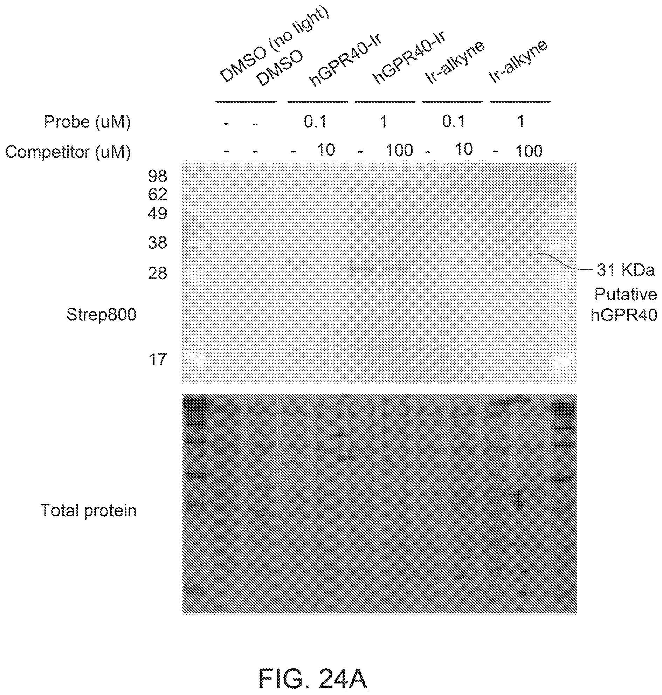

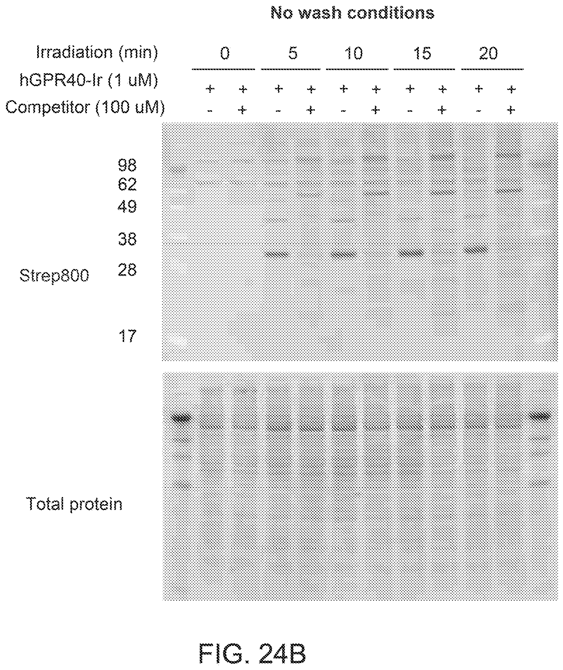

Human GPR40 receptor (hGPR40) was also subjected to labeling with compositions and methods described herein. A hGPR40-Ir conjugate was employed along with a protein labeling agent of biotinylated diazirine (Diaz-PEG3-Bt) (FIG. 23). A hGPR40-ligand was also used as a competitor. HEK-hGPR40 cells were treated with hGPR40-Ir conjugates or free Ir-alkyne photocatalyst for 30 minutes, washed and irradiated for 10 min at 450 nm after treatment with (Diaz-PEG3-Bt). Cell lysates were processed and analyzed by Western blot with Streptavidin-800 and total protein stains, as provided in FIGS. 24A and 24B.

Various embodiments of the invention have been described in fulfillment of the various objects of the invention. It should be recognized that these embodiments are merely illustrative of the principles of the present invention. Numerous modifications and adaptations thereof will be readily apparent to those skilled in the art without departing from the spirit and scope of the invention.

The claims of the parent application are reproduced below as "features" to form basis for future amendment or division as required.

### FEATURES

1. A composition for proximity-based labeling comprising:
   a catalyst; and
   a protein labeling agent, wherein the catalyst has electronic structure to activate the protein labeling agent to a reactive intermediate via energy transfer.
2. The composition of feature 1, wherein the reactive intermediate crosslinks with a protein.
3. The composition of feature 1, wherein the reactive intermediate inserts into a C-H bond of a protein.
4. The composition of feature 1, wherein the reactive intermediate is a carbene, nitrene or phenoxy radical.
5. The composition of feature 1, wherein the reactive intermediate has a diffusion radius less than 4 nm prior to quenching in an aqueous or aqueous-based environment.
6. The composition of feature 1, wherein the reactive intermediate has a half-life (t_{1/2}) less than 5 nanoseconds (ns).
7. The composition of feature 1, wherein the energy transfer is Dexter energy transfer.
8. The composition of feature 1, wherein the energy transfer is single electron transfer.
9. The composition of feature 1, wherein the energy transfer is from a triplet excited state of the catalyst.
10. The composition of feature 9, wherein the catalyst is a photocatalyst.
11. The composition of feature 10, wherein the photocatalyst absorbs light in the visible region of the electromagnetic spectrum.
12. The composition of feature 1, wherein the catalyst is a transition metal photocatalyst having absorption in the visible region of the electromagnetic spectrum.
13. The composition of feature 12, wherein the energy transfer is from a triplet excited state of the photocatalyst, the triplet excited state having energy greater than 60 kcal/mol.
14. The composition of feature 12, wherein the transition metal photocatalyst is of the formula 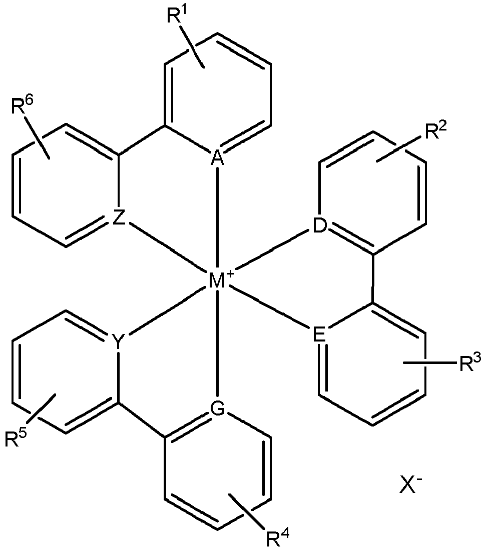
   wherein M is a transition metal;
   wherein A, D, E, G, Y and Z are independently selected from C and N;
   wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
   wherein X⁻ is a counterion.
15. The composition of feature 12, wherein the transition metal photocatalyst is soluble in an aqueous or aqueous-based environment.
16. The composition as in any of features 12 to 15, wherein the protein labeling agent is a diazirine or azide.
17. The composition of feature 1, wherein the catalyst is an organo-photocatalyst.
18. The composition of feature 17, wherein the organo-photocatalyst is selected from group consisting of a thioxanthone, phenolthiazine, flavin, phenoazine, coumarin, acetophenone, and benzophenone group.
19. The composition of feature 18, wherein the energy transfer is single electron transfer.
20. The composition of feature 1, wherein the protein labeling agent is functionalized with a marker, the marker selected from the group consisting of biotin, desthiobiotin, alkyne, azide, FLAG tag, fluorophore, and chloroalkane functionalities.
21. A conjugate comprising:
   a transition metal photocatalyst coupled to a biomolecular binding agent.
22. The conjugate of feature 21, wherein the biomolecular binding agent comprises a protein, polysaccharide, nucleic acid, or lipid.
23. The conjugate of feature 21, wherein the biomolecular binding agent comprises a ligand specific to a cell surface receptor.
24. The conjugate of feature 21, wherein the transition metal photocatalyst has a triplet excited state of greater than 60 kcal/mol.
25. The conjugate as in any of features 21 to 24, wherein the transition metal complex is of the formula 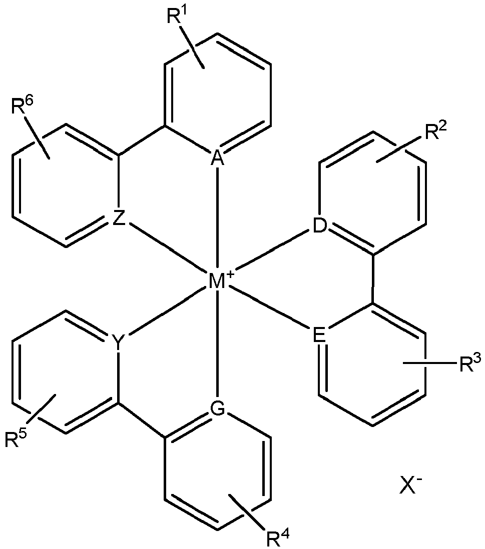
   wherein M is a transition metal;
   wherein A, D, E, G, Y and Z are independently selected from C and N;
   wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
   wherein X⁻ is a counterion.
26. The conjugate as in any of features 21 to 24 further comprising a hydrophilic linker between the transition metal photocatalyst and the biomolecular binding agent.
27. The conjugate of feature 26, wherein the linker comprises alkylene oxide.
28. The conjugate of feature 21, wherein the transition metal photocatalyst absorb light in the visible region of the electromagnetic spectrum.
29. The conjugate of feature 21, wherein the biomolecular binding agent is covalently bound to the transition metal photocatalyst.
30. A system for proximity-based labeling comprising:
   a conjugate including a catalyst coupled to a biomolecular binding agent; and
   a protein labeling agent, wherein the catalyst has electronic structure to activate the protein labeling agent to a reactive intermediate via energy transfer.
31. The system of feature 30, wherein the reactive intermediate crosslinks with a protein.
32. The system of feature 30, wherein the biomolecular binding agent comprises a ligand specific to a cell surface receptor, and the reactive intermediate crosslinks with the surface cell receptor.
33. The system of feature 30, wherein the reactive intermediate inserts into a C-H bond of a protein.
34. The system of feature 33, wherein the reactive intermediate is a carbene.
35. The system of feature 30, wherein the reactive intermediate has a diffusion radius less than 4 nm prior to quenching in an aqueous or aqueous-based environment.
36. The system of feature 30, wherein the energy transfer is Dexter energy transfer.
37. The system of feature 30, wherein the energy transfer is single electron transfer.
38. The system of feature 30, wherein the energy transfer is from a triplet excited state of the catalyst.
39. The system of feature 38, wherein the catalyst absorbs light in the visible region of the electromagnetic spectrum.
40. The system of feature 30, wherein the catalyst is a transition metal photocatalyst having absorption in the visible region of the electromagnetic spectrum.
41. The system of feature 40, wherein the energy transfer is from a triplet excited state of the photocatalyst, the triplet state having energy greater than 60 kcal/mol.
42. The system of feature 40 or 41, wherein the transition metal photocatalyst is of the formula 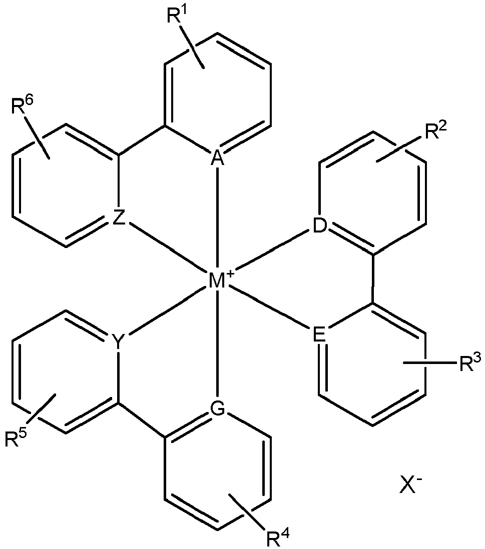
   wherein M is a transition metal;
   wherein A, D, E, G, Y and Z are independently selected from C and N;
   wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
   wherein X⁻ is a counterion.
43. The system of feature 30, wherein the biomolecular binding agent comprises a protein, polysaccharide, nucleic acid, or lipid.
44. The system of feature 40 or feature 41, wherein the protein labeling agent is a diazirine or azide.
45. A method of proximity-based labeling comprising:
   providing a conjugate comprising a catalyst coupled to a biomolecular binding agent;
   locating the conjugate in a targeted area of a cellular membrane with the biomolecular binding agent;
   activating a protein labeling agent to a reactive intermediate with the catalyst; and
   coupling the reactive intermediate to one or more proteins in the targeted area.
46. The method of feature 45, wherein the biomolecular binding agent comprises a ligand specific to a cell surface receptor.
47. The method of feature 45, wherein the reactive intermediate inserts into a C-H bond of the one or more proteins.
48. The method of feature 45, wherein the reactive intermediate has a diffusion radius less than 4 nm prior to quenching in an aqueous or aqueous-based environment.
49. The method of feature 45, wherein the catalyst has electronic structure to activate the protein labeling agent to the reactive intermediate via energy transfer.
50. The method of feature 49, wherein the energy transfer is Dexter energy transfer.
51. The method of feature 49, wherein the energy transfer is single electron transfer.
52. The method of feature 49, wherein the energy transfer is from a triplet excited state of the catalyst.
53. The method of feature 52, wherein the excited triplet state has energy greater than 60 kcal/mol.
54. The method as in any of features 49 to 53, wherein the catalyst is a transition metal photocatalyst.
55. The method of feature 54, wherein the transition metal photocatalyst is of the formula 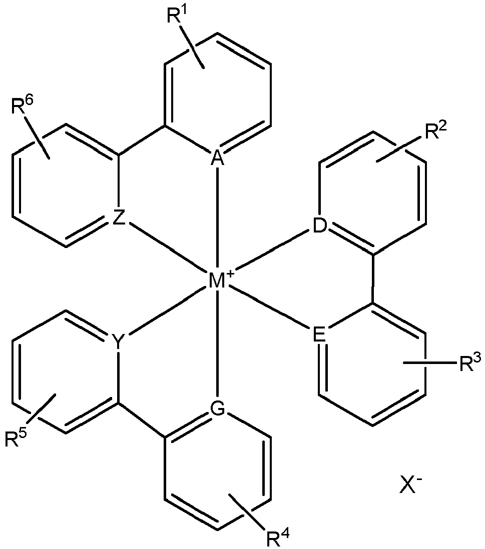
   wherein M is a transition metal;
   wherein A, D, E, G, Y and Z are independently selected from C and N;
   wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
   wherein X⁻ is a counterion.
56. The method of feature 54, wherein the protein labeling agent is a diazirine or azide.
57. The method of feature 45 further comprising enriching the one or more proteins coupled to the reactive intermediate.
58. The method of feature 45, wherein the biomolecular binding agent comprises a protein, polysaccharide, nucleic acid, or lipid.
59. The method of feature 45, wherein the one or more proteins are associated with the cellular membrane.
60. The method of feature 45, wherein the one or more proteins are associated with an adjacent cell in contact with the cellular membrane.
61. The method of feature 45 further comprising identifying the one or more proteins coupled to the reactive intermediate for interactome mapping.
62. The method of feature 45 further comprising purifying one or more proteins coupled to the reactive intermediate.
63. The method of feature 62, wherein purifying comprises affinity chromatography.
64. The method of feature 62 further comprising purifying one or more proteins in the targeted area not labeled with the protein labeling agent.
65. The method of feature 45, wherein the one or more proteins coupled by the reactive intermediate are identifiable in the targeted area via microscopy.

## Claims

1. A composition for proximity-based labeling comprising:
a photocatalyst; and
a protein labeling agent, wherein the photocatalyst has electronic structure to activate the protein labeling agent to a reactive intermediate via energy transfer, and wherein the reactive intermediate is a carbene, nitrene or phenoxy radical.

2. The composition of claim 1, wherein the reactive intermediate is a carbene.

3. The composition of claim 1, wherein the reactive intermediate has a diffusion radius less than 4 nm prior to quenching in an aqueous or aqueous-based environment.

4. The composition of claim 1, wherein the reactive intermediate has a half-life (t_{1/2}) less than 5 nanoseconds (ns).

5. The composition of claim 1, wherein the energy transfer is Dexter energy transfer.

6. The composition of claim 1, wherein the energy transfer is single electron transfer.

7. The composition of claim 1, wherein the energy transfer is from a triplet excited state of the catalyst.

8. The composition of claim 1, wherein the photocatalyst absorbs light in the visible region of the electromagnetic spectrum.

9. The composition of claim 1, wherein the catalyst is a transition metal photocatalyst.

10. The composition of claim 9, wherein the energy transfer is from a triplet excited state of the photocatalyst, the triplet excited state having energy greater than 60 kcal/mol (~250kJ/mol).

11. The composition of claim 9, wherein the transition metal photocatalyst is of the formula 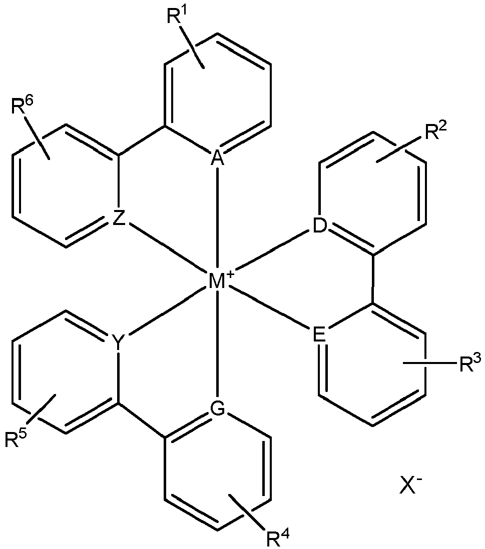
wherein M is a transition metal;
wherein A, D, E, G, Y and Z are independently selected from C and N;
wherein R¹ - R⁶ each represent one to four optional ring substituents, each of the one to four optional ring substituents independently selected from the group consisting of alkyl, heteroalkyl, haloalkyl, halo, hydroxy, alkoxy, amine, amide, ether, -C(O)O⁻, -C(O)OR⁷, and -R⁸OH, wherein R⁷ is selected from the group consisting of hydrogen and alkyl, and R⁸ is alkyl; and
wherein X⁻ is a counterion.

12. The composition as in any of claims 9 to 11, wherein the protein labeling agent is a diazirine or azide.

13. The composition of claim 1, wherein the catalyst is an organo-photocatalyst.

14. The composition of claim 13, wherein the organo-photocatalyst is selected from group consisting of a thioxanthone, phenolthiazine, flavin, phenoazine, coumarin, acetophenone, and benzophenone group.

15. The composition of claim 14, wherein the energy transfer is single electron transfer.

16. The composition of claim 1, further comprising a biomolecular binding agent coupled to the photocatalyst.

17. The composition of claim 16, wherein the biomolecular binding agent comprises a protein, polysaccharide, nucleic acid, or lipid.

18. The composition of claim 16, wherein the biomolecular binding agent comprises a ligand specific to a cell surface receptor.

19. The composition of claim 16, wherein the photocatalyst is a transition metal photocatalyst, and wherein the biomolecular binding agent is covalently bound to the transition metal photocatalyst.
